# EUROPEAN PATENT SPECIFICATION

(11) **EP 3 426 636 B1**
(45) Date of publication and mention of the grant of the patent: **29.12.2021**
(21) Application number: 17763867.3
(22) Date of filing: 07.03.2017
(51) Int. Cl.: C07D 231/18, C07D 231/12, C07D 231/14, C07D 231/16, C07D 401/04, C07D 401/12, C07D 401/14, C07D 403/04, C07D 405/04, C07D 405/12, C07D 498/04, C07D 239/28, C07D 239/42, C07D 249/06, C07D 261/08, A61K 31/415, A61K 31/4155, A61K 31/4439, A61K 31/496, A61K 31/497, A61K 31/5377, A61P 35/00

(54) **SPECIFIC 2-(4-(PHENOXY)-1H-PYRAZOL-3-YL)PHENOL DERIVATIVES AS INHIBITORS OF C-MYC/DNA BINDING ACTIVITY FOR TREATING CANCER**
SPEZIFISCHE 2-(4-(PHENOXY)-1H-PYRAZOL-3-YL)PHENOLDERIVATE ALS INHIBITOREN DER C-MYC/DNA BINDUNGSAKTIVITÄT ZUR BEHANDLUNG VON KREBS
DÉRIVÉS SPECIFIQUES DE 2-(4-(PHÉNOXY)-1H-PYRAZOL-3-YL)PHÉNOL EN TANT QU'INHIBITEURS DE L'ACTIVITÉ DE CIBLAGE DE C-MYC/DNA POUR LE TRAITEMENT DU CANCER

(30) Priority: 07.03.2016 US 201662304704 P
(43) Date of publication of application: 16.01.2019
(73) Proprietor: Northwestern University, Evanston, IL 60208 (US)
(72) Inventor: SCHILTZ, Gary E., Naperville, IL 60565 (US); MISHRA, Rama K., Chicago, IL 60607 (US); HAN, Huiying, Chicago, IL 60616 (US); ABDULKADIR, Sarki A., Lombard, IL 60148 (US); IZQUIERDO-FERRER, Javier, Chicago, IL 60611 (US); JAIN, Atul D., Chicago, IL 60640 (US)
(74) Representative: Boult Wade Tennant LLP
(86) International application number: PCT/US2017/021082
(87) International publication number: WO 2017/155942

(56) References cited:
- EP-A1- 1 108 720
- EP-A1- 2 738 172
- WO-A1-2006/018082
- WO-A1-2007/136703
- WO-A2-2006/079057
- WO-A2-2010/111653
- WO-A2-2014/059429
- DE-A1- 2 551 868
- US-A- 3 414 582
- US-A1- 2008 090 880
- US-A1- 2012 316 182
- US-A1- 2013 005 666
- MATIN AZADEH ET AL.: '7- Hydroxy-benzopyran-4-one Derivatives: A Novel Pharmacophore of Peroxisome Proliferator-Activated Receptor alpha and -y (PPARa and y) Dual Agonists' JOURNAL OF MEDICAL CHEMISTRY vol. 52, 2009, pages 6835 - 6850, XP055384761
- SOSNOVSKIKH V. YA. ET AL.: '2-Polyfluoroalkylchromones 1 l.*Synthesis and structure of 5-hydroxy-3-(2-hydroxyaryl)-5-polyfluoroalk yl-DELTA2-pyrazolines and 3(5)- (2-hydroxyaryl)-5(3)- polyfluoroalkylpyrazoles' RUSSIAN CHEMICAL BULLETIN, INTERNATIONAL EDITION vol. 51, no. 7, 2002, pages 1280 - 1291, XP002595193
- SAPEGIN ALEXANDER V. ET AL.: 'New tetracyclic 1,4-oxazepines constructed via practically simple tandem condensation strategy from readily available synthons' TETRAHEDRON vol. 70, 2014, pages 1077 - 1083, XP028808756
- PARK HWANGSEO ET AL.: 'A novel class of Hsp90 inhibitors isolated by structure-based virtual screening' BIOORGANIC & MEDICINAL CHEMISTRY LETTERS vol. 17, 2007, pages 6345 - 6349, XP022297156
- DATABASE DATABASE [Online] 01 May 2015 STN, XP055431544 Database accession no. 1695913-02-0
- DATABASE DATABASE 09 February 2014 STN, XP055431549 Database accession no. 11539648-50-4
- DATABASE DATABASE 21 June 2015 STN, XP055431552 Database accession no. 1785193-21-6
- DATABASE DATABASE 25 February 2014 STN, XP055431556 Database accession no. 1555124-94-1
- DATABASE DATABASE 28 January 2014 STN, XP055431558 Database accession no. 1532498-32-0

## Description

### BACKGROUND

The field of the invention relates to substituted heterocycles as c-MYC targeting agents. In particular, the field of the invention relates to substituted pyrazoles as c-MYC targeting agents for the treatment of cell proliferation diseases and disorders such as cancer.

The c-MYC oncogene is de-regulated and plays a causal role in a majority of human cancer and c-MYC inhibition profoundly affects tumor growth or survival in multiple models. *MYC* is the most common oncogene involved in human cancers and is overexpressed in up to half of all cancers. Therefore, developing c-MYC inhibitors is among the most attractive potential anti-cancer strategies. Unfortunately, due to the difficulty in targeting transcription factors with small molecules, c-MYC is currently regarded as "undruggable." Here, we disclose a new approach to targeting c-MYC and have developed a series of new small molecule inhibitors. These compounds selectively target c-MYC-driven cell proliferation and interfere with binding of c-MYC to DNA.

Pyrazole containing compounds are disclosed in US2008/090880, WO2006/018082, US2013/005666 and WO2010/111653.

### SUMMARY

The invention is defined in the appended claims.

Disclosed are substituted heterocycles which may be utilizes as c-MYC targeting agents. The substituted heterocycles may include substituted pyrazoles. The disclosed heterocycles may be used in pharmaceutical compositions and methods for treating cell proliferative disorders such as cancer.

The disclosed compounds may exhibit one or more biological activities. The disclosed compounds may inhibit binding of the Myc/Max complex to DNA (*e.g.,* in a DNA gel shifting assay). The disclosed compounds may not produce significant DNA damage (*e.g.,* in an rH2AX staining assay at a concentration greater than about 0.001 µM, 0.005 µM , 0.01 µM, 0.1 µM, 1.0 µM, 10 µM, 100 µM, or higher). The disclosed compounds may inhibit the growth of cells that express c-Myc (preferably by at a concentration of less than about 100 µM, 50 µM, 10 µM, 1 µM, 0.1 µM, 0.05 µM, 0.01 µM, 0.005 µM, 0.001 µM, or less). The disclosed compounds may not inhibit the growth of cells that do not express c-Myc (preferably at a concentration of greater than about 0.001 µM, 0.005 µM, 0.01 µM, 0.5 µM, 0.1 µM, 1.0 µM, 10 µM, and 100 µM or higher).

Also disclosed are pharmaceutical compositions comprising the disclosed compounds and a suitable pharmaceutical carrier, excipient, or diluent. The disclosed pharmaceutical compositions may comprise an effective amount of the compound for inhibiting the growth of cancer cells when administered to a subject in need thereof.

Also disclosed are methods for treating cell proliferation diseases and disorders such as cancer. The methods may include administering the disclosed compounds or pharmaceutical compositions comprising the disclosed compounds to a subject in need thereof, for example, to a subject having cancer. Cell proliferative diseases and disorders treated by the disclosed methods may include, but are not limited to, cancers selected from the group consisting of multiple myeloma, leukemia, non-small cell lung cancer, colon cancer, cancer of the central nervous system, melanoma, ovarian cancer, renal cancer, prostate cancer, and breast cancer.

### BRIEF DESCRIPTION OF THE FIGURES

Figure 1. Scheme for in silico screen to identify c-Myc inhibitors.
Figure 2. Relative Myc E-box luciferase inhibitory activity of 32 compounds.
Figure 3. Growth inhibition by selected hit compound on Myc WT and KO fibroblasts.
Figure 4. Cell viability after treatment with Min9 (NUCC-0176234).
Figure 5. Electrophoretic mobility shift assay (EMSA) in the presence of 200 µM test compounds.
Figure 6. (B) Relative values of DNA bound for test compounds at 200 µM.

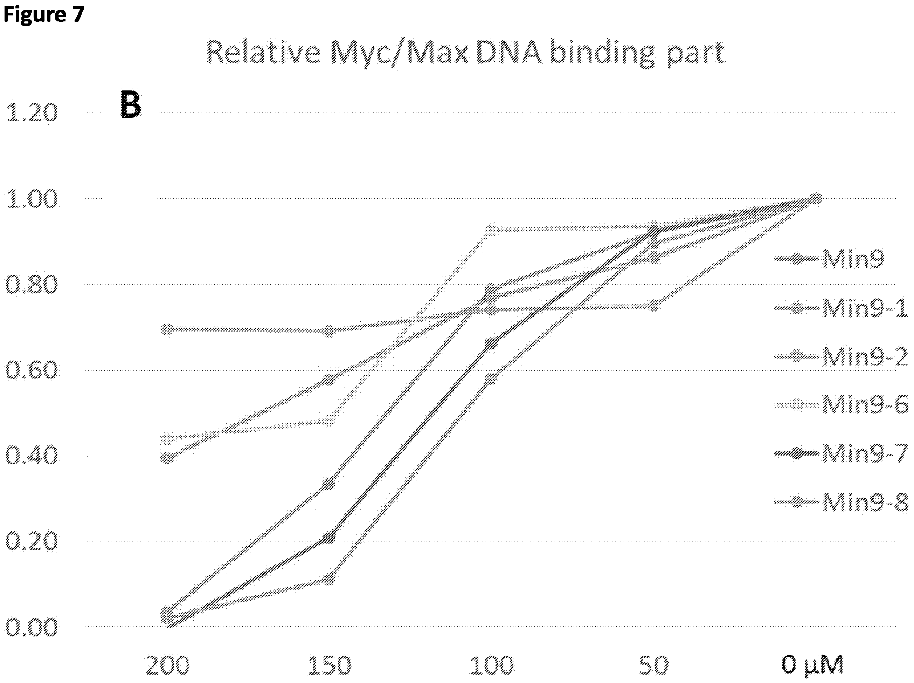

Figure 7. (C) Relative Myc/Max DNA binding versus concentration of compound.

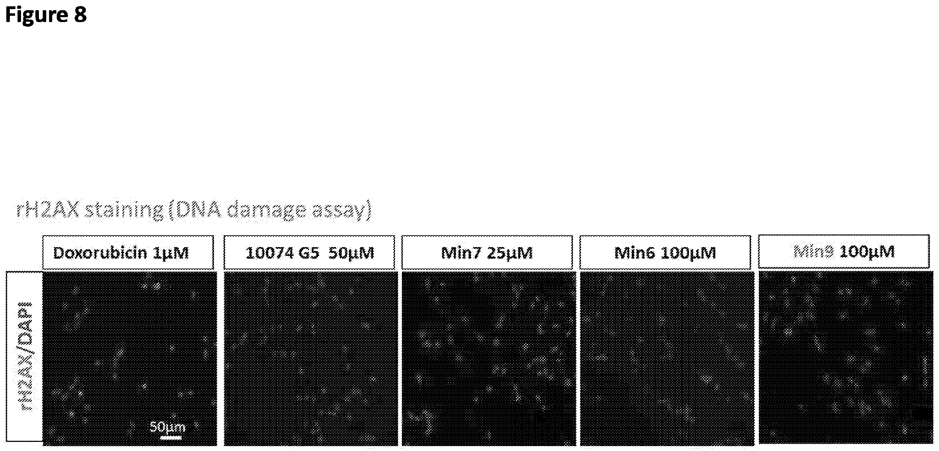

Figure 8. rH2AX assay for DNA damage.
Figure 9. Scheme 1 for synthesis of new substituted pyrazole derivatives.
Figure 10. Scheme 2 for synthesis of diverse analogs of Min9 (NUCC-0176234) such as Min9-S7 (NUCC-176248).
Figure 11. Scheme 3 for synthesis of substituted chromenones.

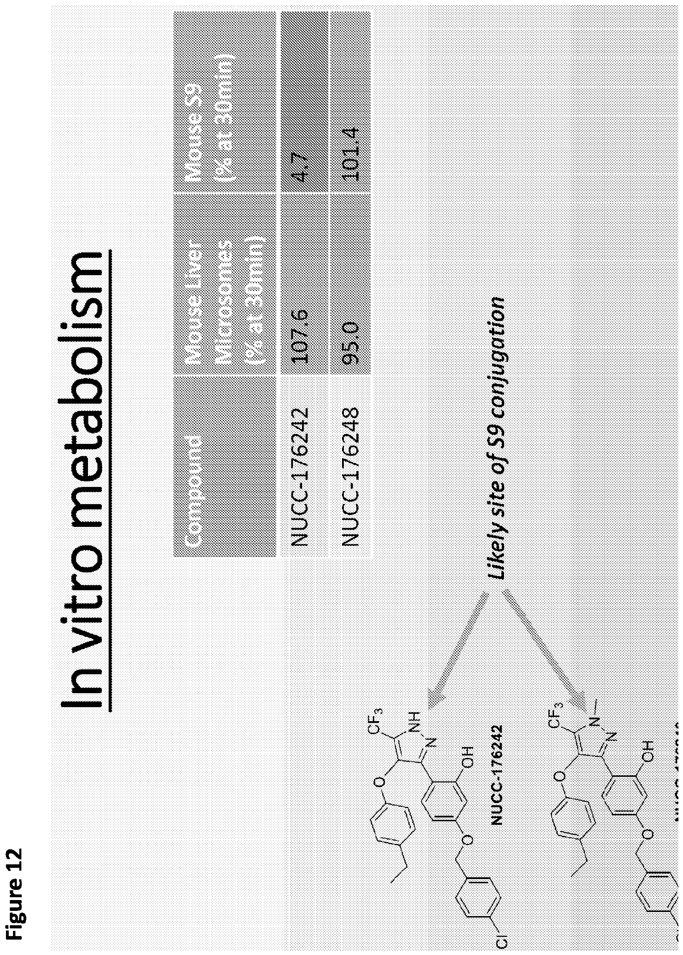

Figure 12. In vitro metabolism of NUCC-176242 versus NUCC-176248.

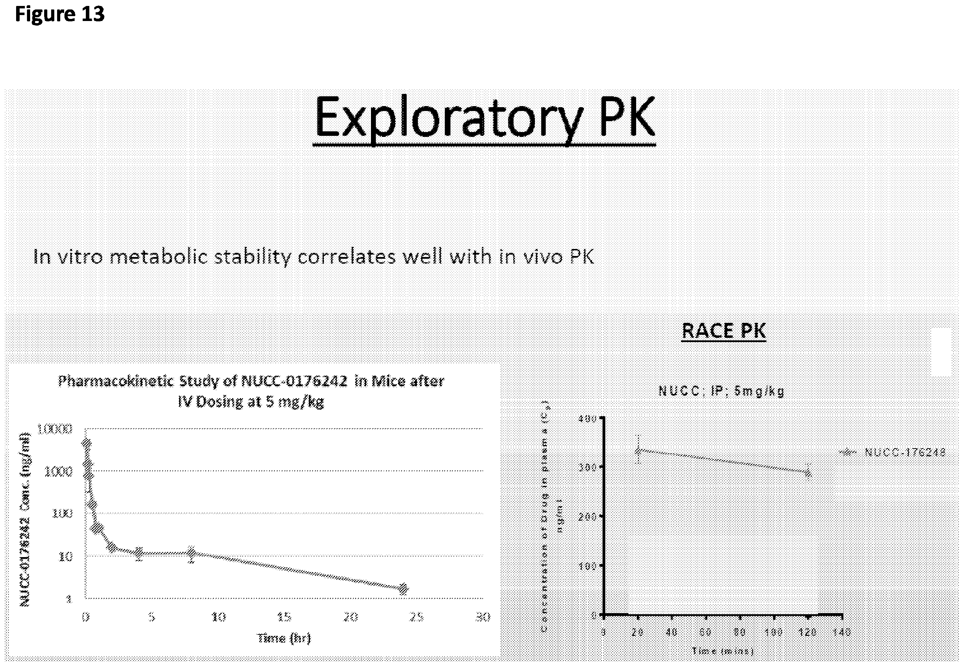

Figure 13. Pharmacokinetic study of NUCC-176242 in mice after IV dosing at 5 mg/kg.

### DETAILED DESCRIPTION

The present invention is described herein using several definitions, as set forth below and throughout the application.

Unless otherwise specified or indicated by context, the terms "a", "an", and "the" mean "one or more." For example, "a compound" should be interpreted to mean "one or more compounds."

As used herein, "about," "approximately," "substantially," and "significantly" will be understood by persons of ordinary skill in the art and will vary to some extent on the context in which they are used. If there are uses of these terms which are not clear to persons of ordinary skill in the art given the context in which they are used, "about" and "approximately" will mean plus or minus ≤10% of the particular term and "substantially" and "significantly" will mean plus or minus > 10% of the particular term.

As used herein, the terms "include" and "including" have the same meaning as the terms "comprise" and "comprising" in that these latter terms are "open" transitional terms that do not limit claims only to the recited elements succeeding these transitional terms. The term "consisting of," while encompassed by the term "comprising," should be interpreted as a "closed" transitional term that limits claims only to the recited elements succeeding this transitional term. The term "consisting essentially of," while encompassed by the term "comprising," should be interpreted as a "partially closed" transitional term which permits additional elements succeeding this transitional term, but only if those additional elements do not materially affect the basic and novel characteristics of the claim.

As used herein, a "subject" may be interchangeable with "patient" or "individual" and means an animal, which may be a human or non-human animal, in need of treatment.

A "subject in need of treatment" may include a subject having a disease, disorder, or condition that is responsive to therapy with a substituted heterocycle such as the presently disclosed substituted pyrazoles, substituted pyrimidines, and substituted triazoles. For example, a "subject in need of treatment" may include a subject having a cell proliferative disease, disorder, or condition such as cancer (*e.g.,* cancers such as multiple myeloma, leukemia, non-small cell lung cancer, colon cancer, cancer of the central nervous system, melanoma, ovarian cancer, renal cancer, prostate cancer, and breast cancer).

As used herein, the phrase "effective amount" shall mean that drug dosage that provides the specific pharmacological response for which the drug is administered in a significant number of subject in need of such treatment. An effective amount of a drug that is administered to a particular subject in a particular instance will not always be effective in treating the conditions/diseases described herein, even though such dosage is deemed to be a therapeutically effective amount by those of skill in the art.

Disclosed herein are substituted heterocycles. The disclosed heterocycles have been shown to inhibit the biological activity of c-Myc.

The formulae of the compounds disclosed herein should be interpreted as encompassing all possible stereoisomers, enantiomers, or epimers of the compounds unless the formulae indicates a specific stereoisomer, enantiomer, or epimer. The formulae of the compounds disclosed herein should be interpreted as encompassing salts, esters, amides, or solvates thereof of the compounds.

The disclosed compounds may exhibit one or more biological activities. The disclosed compounds may inhibit binding of the Myc/Max complex to DNA (*e.g.,* in a DNA gel shifting assay). In some embodiments, the disclosed compounds inhibit binding of the Myc/Max complex to DNA by at least 50%, 60%, 70%, 80%, 90%, 95%, 96%, 97%, 98%, or 99% at a concentration of less than about 100 µM, 50 µM, 10 µM, 1 µM, 0.1 µM, 0.05 µM, 0.01 µM, 0.005 µM, 0.001 µM, or less. The disclosed compounds may not produce significant DNA damage (*e.g.,* in an rH2AX staining assay at a concentration greater than about 0.001 µM, 0.005 µM , 0.01 µM, 0.1 µM, 1.0 µM, 10 µM, 100 µM, or higher). The disclosed compounds may inhibit the growth of cells that express c-Myc (preferably by at least 50%, 60%, 70%, 80%, 90%, 95%, 96%, 97%, 98%, or 99% at a concentration of less than about 100 µM, 50 µM, 10 µM, 1 µM, 0.1 µM, 0.05 µM, 0.01 µM, 0.005 µM, 0.001 µM, or less). The disclosed compounds may not inhibit the growth of cells that do not express c-Myc (preferably by not more than 50%, 40%, 30%, 20%, 10%, 5%, 4%, 3%, 2% or less at a concentration of greater than about 0.001 µM, 0.005 µM, 0.01 µM, 0.5 µM, 0.1 µM, 1.0 µM, 10 µM, and 100 µM or higher). Concentration ranges also are contemplated herein, for example, a concentration range bounded by end-point concentrations selected from 0.001 µM, 0.005 µM, 0.01 µM, 0.5 µM, 0.1 µM, 1.0 µM, 10 µM, and 100 µM.

The disclosed compounds may be effective in inhibiting cell proliferation of cancer cells, including cancer cells that express c-MYC and whose proliferation is inhibiting by inhibiting the biological activity of c-MYC. The disclosed compounds may be effective in inhibiting cell proliferation of one or more types of cancer cells including: multiple myeloma cells, such as MM.1S cells; leukemia cells, such as CCRF-CEM, HL-60(TB), MOLT-4, RPMI-8226 and SR; non-small lung cancer cells, such as A549/ATCC, EKVX, HOP-62, HOP-92, NCI-H226, NCI-H23, NCI-H322M, NCI-H460 and NCI-H522; colon cancer cells, such as COLO 205, HCC-2998, HCT-116, HCT-15, HT29, KM12 and SW-620; CNS: SF-268, SF-295, SF-539, SNB-19, SNB-75 and U251; melanoma cancer cells, such as LOX IMVI, MALME-3M, M14, MDA-MB-435, SK-MEL-2, SK-MEL-28, SK-MEL-5, UACC-257 and UACC-62; ovarian cancer cells, such as IGR-OV1, OVCAR-3, OVCAR-4, OVCAR-5, OVCAR-8, NCI/ADR-RES and SK-OV-3; renal cancer cells, such as 786-0, A498, ACHN, CAKI-1, RXF 393, SN12C, TK-10 and UO-31; prostate cancer cells, such as DU-145 and PC-3; and breast cancer cells, such as MCF7, MDA-MB-231/ATCC, MDA-MB-468, HS 578T, BT-549 and T-47D.

Cell proliferation and inhibition thereof by the presently disclosed compounds may be assessed by cell viability methods disclosed in the art including colorimetric assays that utilize dyes such as MTT, XTT, and MTS to assess cell viability. Preferably, the disclosed compounds have an IC₅₀ of less than about 10 µM, 5 µM, 1 µM, 0.5 µM, 0.01 µM, 0.005 µM, 0.001 µM or lower in the selected assay.

The disclosed compounds may be formulated as anti-cancer therapeutics, including hematologic malignancies, breast, lung, pancreas and prostate malignancies. The disclosed compounds also may be formulated as anti-inflammation therapeutics.

The compounds utilized in the methods disclosed herein may be formulated as pharmaceutical compositions that include: (a) a therapeutically effective amount of one or more compounds as disclosed herein; and (b) one or more pharmaceutically acceptable carriers, excipients, or diluents. The pharmaceutical composition may include the compound in a range of about 0.1 to 2000 mg (preferably about 0.5 to 500 mg, and more preferably about 1 to 100 mg). The pharmaceutical composition may be administered to provide the compound at a daily dose of about 0.1 to 100 mg/kg body weight (preferably about 0.5 to 20 mg/kg body weight, more preferably about 0.1 to 10 mg/kg body weight). In some embodiments, after the pharmaceutical composition is administered to a subject *(e.g.,* after about 1, 2, 3, 4, 5, or 6 hours post-administration), the concentration of the compound at the site of action may be within a concentration range bounded by end-points selected from 0.001 µM, 0.005 µM, 0.01 µM, 0.5 µM, 0.1 µM, 1.0 µM, 10 µM, and 100 µM (*e.g.,* 0.1 µM - 1.0 µM).

The disclosed compounds and pharmaceutical compositions comprising the disclosed compounds may be administered in methods of treating a subject in need thereof. For example, in the methods of treatment a subject in need thereof may include a subject having a cell proliferative disease, disorder, or condition such as cancer (*e.g.,* cancers such as multiple myeloma, leukemia, non-small cell lung cancer, colon cancer, cancer of the central nervous system, melanoma, ovarian cancer, renal cancer, prostate cancer, and breast cancer). Thus, the invention relates to pharmaceutical compositions comprising the disclosed compounds for use in a method of treating cancer.

In some embodiments of the medical uses, the subject may be administered a dose of a compound as low as 1.25 mg, 2.5 mg, 5 mg, 7.5 mg, 10 mg, 12.5 mg, 15 mg, 17.5 mg, 20 mg, 22.5 mg, 25 mg, 27.5 mg, 30 mg, 32.5 mg, 35 mg, 37.5 mg, 40 mg, 42.5 mg, 45 mg, 47.5 mg, 50 mg, 52.5 mg, 55 mg, 57.5 mg, 60 mg, 62.5 mg, 65 mg, 67.5 mg, 70 mg, 72.5 mg, 75 mg, 77.5 mg, 80 mg, 82.5 mg, 85 mg, 87.5 mg, 90 mg, 100 mg, 200 mg, 500 mg, 1000 mg, or 2000 mg once daily, twice daily, three times daily, four times daily, once weekly, twice weekly, or three times per week in order to treat the disease or disorder in the subject. In some embodiments, the subject may be administered a dose of a compound as high as 1.25 mg, 2.5 mg, 5 mg, 7.5 mg, 10 mg, 12.5 mg, 15 mg, 17.5 mg, 20 mg, 22.5 mg, 25 mg, 27.5 mg, 30 mg, 32.5 mg, 35 mg, 37.5 mg, 40 mg, 42.5 mg, 45 mg, 47.5 mg, 50 mg, 52.5 mg, 55 mg, 57.5 mg, 60 mg, 62.5 mg, 65 mg, 67.5 mg, 70 mg, 72.5 mg, 75 mg, 77.5 mg, 80 mg, 82.5 mg, 85 mg, 87.5 mg, 90 mg, 100 mg, 200 mg, 500 mg, 1000 mg, or 2000 mg, once daily, twice daily, three times daily, four times daily, once weekly, twice weekly, or three times per week in order to treat the disease or disorder in the subject. Minimal and/or maximal doses of the compounds may include doses falling within dose ranges having as end-points any of these disclosed doses (*e.g.,* 2.5 mg - 200 mg).

In some embodiments, a minimal dose level of a compound for achieving therapy may be at least about 10, 20, 30, 40, 50, 60, 70, 80, 90, 100, 150, 200, 250, 300, 350, 400, 450, 500, 550, 600, 650, 700, 750, 800, 850, 900, 950, 1000, 1200, 1400, 1600, 1800, 1900, 2000, 3000, 4000, 5000, 6000, 7000, 8000, 9000, 10000, 15000, or 20000 ng/kg body weight of the subject. In some embodiments, a maximal dose level of a compound for achieving therapy may not exceed about 10, 20, 30, 40, 50, 60, 70, 80, 90, 100, 150, 200, 250, 300, 350, 400, 450, 500, 550, 600, 650, 700, 750, 800, 850, 900, 950, 1000, 1200, 1400, 1600, 1800, 1900, 2000, 3000, 4000, 5000, 6000, 7000, 8000, 9000, 10000, 15000, or 20000 ng/kg body weight of the subject. Minimal and/or maximal dose levels of the compounds for achieving therapy may include dose levels falling within ranges having as end-points any of these disclosed dose levels (*e.g.,* 500 - 2000 ng/kg body weight of the subject).

The compounds utilized in the methods disclosed herein may be formulated as a pharmaceutical composition in solid dosage form, although any pharmaceutically acceptable dosage form can be utilized. Exemplary solid dosage forms include, but are not limited to, tablets, capsules, sachets, lozenges, powders, pills, or granules, and the solid dosage form can be, for example, a fast melt dosage form, controlled release dosage form, lyophilized dosage form, delayed release dosage form, extended release dosage form, pulsatile release dosage form, mixed immediate release and controlled release dosage form, or a combination thereof.

The compounds utilized in the methods disclosed herein may be formulated as a pharmaceutical composition that includes a carrier. For example, the carrier may be selected from the group consisting of proteins, carbohydrates, sugar, talc, magnesium stearate, cellulose, calcium carbonate, and starch-gelatin paste.

The compounds utilized in the methods disclosed herein may be formulated as a pharmaceutical composition that includes one or more binding agents, filling agents, lubricating agents, suspending agents, sweeteners, flavoring agents, preservatives, buffers, wetting agents, disintegrants, and effervescent agents. Filling agents may include lactose monohydrate, lactose anhydrous, and various starches; examples of binding agents are various celluloses and crosslinked polyvinylpyrrolidone, microcrystalline cellulose, such as Avicel^{®} PH101 and Avicel^{®} PH102, microcrystalline cellulose, and silicified microcrystalline cellulose (ProSolv SMCC^{™}). Suitable lubricants, including agents that act on the flowability of the powder to be compressed, may include colloidal silicon dioxide, such as Aerosil^{®}200, talc, stearic acid, magnesium stearate, calcium stearate, and silica gel. Examples of sweeteners may include any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like. Examples of preservatives may include potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quaternary compounds such as benzalkonium chloride.

Suitable diluents may include pharmaceutically acceptable inert fillers, such as microcrystalline cellulose, lactose, dibasic calcium phosphate, saccharides, and mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

The compounds disclosed herein may be formulated as a pharmaceutical composition for delivery via any suitable route. For example, the pharmaceutical composition may be administered via oral, intravenous, intramuscular, subcutaneous, topical, and pulmonary route. Examples of pharmaceutical compositions for oral administration include capsules, syrups, concentrates, powders and granules.

The compounds disclosed herein may be administered in conventional dosage forms prepared by combining the active ingredient with standard pharmaceutical carriers or diluents according to conventional procedures well known in the art. These procedures may involve mixing, granulating and compressing or dissolving the ingredients as appropriate to the desired preparation.

Pharmaceutical compositions comprising the compounds may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical compositions adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis.

Pharmaceutical compositions adapted for topical administration may be formulated as ointments, creams, suspensions, lotions, powders, solutions, pastes, gels, impregnated dressings, sprays, aerosols or oils and may contain appropriate conventional additives such as preservatives, solvents to assist drug penetration and emollients in ointments and creams.

For applications to the eye or other external tissues, for example the mouth and skin, the pharmaceutical compositions are preferably applied as a topical ointment or cream. When formulated in an ointment, the compound may be employed with either a paraffinic or a water-miscible ointment base. Alternatively, the compound may be formulated in a cream with an oil-in-water cream base or a water-in-oil base. Pharmaceutical compositions adapted for topical administration to the eye include eye drops where the active ingredient is dissolved or suspended in a suitable carrier, especially an aqueous solvent.

Pharmaceutical compositions adapted for nasal administration where the carrier is a solid include a coarse powder having a particle size (*e.g.,* in the range 20 to 500 microns) which is administered in the manner in which snuff is taken (*i.e.,* by rapid inhalation through the nasal passage from a container of the powder held close up to the nose). Suitable formulations where the carrier is a liquid, for administration as a nasal spray or as nasal drops, include aqueous or oil solutions of the active ingredient.

Pharmaceutical compositions adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavoring or coloring agents.

### EXAMPLES

### Example 1 - Identification of Small Molecule Inhibitors of C-Myc DNA Binding Activity

### Introduction

*MYC* is the most frequently amplified oncogene in human cancers. It has been extensively validated as essential for tumor initiation and maintenance in numerous tumor histologies. Numerous studies have provided solid evidence that pharmacologic targeting of Myc would directly affect tumor progression. One example is Omomyc, a dominant-negative peptide of Myc that competitively binds Myc in a manner that prevents Myc-Max heterodimerization. Omomyc expression prompts rapid growth arrest and down-regulation of Myc target genes in cancer cells both in vitro and in vivo. Small molecule inhibitors of Myc will be the optimal form for drug development. However, disruption of Myc-Max interactions through small molecules has been difficult because there are no obvious binding regions in the interface. Thus far, over 30 small molecules have been documented with Myc inhibition activity in vitro, but the evidence for their in vivo activities is lacking, likely due to their poor drug-like properties. Among these compounds, 10058-f4 and 10075-G5 are well-known for their specificities and relatively clear mechanisms in interrupting Myc-Max binding. However, the in vivo studies were quite disappointing because of their rapid metabolism. Thus, developing new Myc inhibitors with high potency and specificity as well as favorable drug-like properties will be critical to effectively target Myc.

To this end, we carried out an in silico screen to identify compounds that might inhibit the binding of c-MYC to DNA. These compounds were tested in several cell-based assays to identify the most active hits. The best hit, Min-9 (NUCC-176234) and its related analogs were shown to prevent c-MYC/DNA binding. We then synthesized a series of novel structural analogs and these were tested in the same c-MYC-relevant assays. *(See* Table 1). Our new compounds display excellent potency at inhibiting c-MYC/DNA binding. The compounds we have developed using a novel approach possess greatly improved drug-like properties over existing small molecules such as 10058-f4 and therefore represent excellent starting points for developing Myc-targeting therapeutics.

### Results

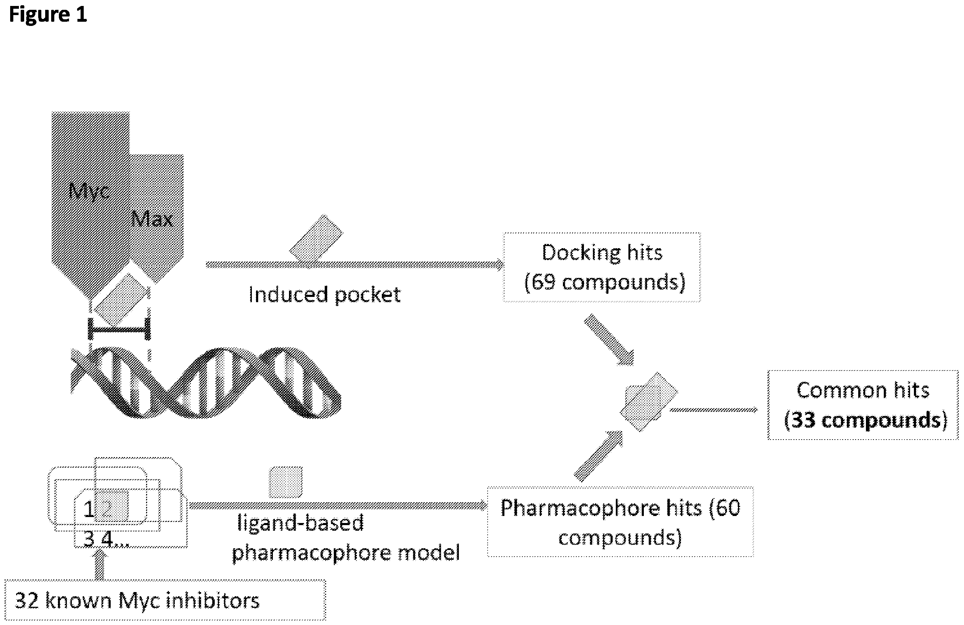

In the absence of a regular small-molecule ligand-binding pocket in the c-Myc/Max/DNA ternary complex, we applied multiple independent *in silico* approaches to increase our likelihood of successfully identifying new small molecule inhibitors. (*See* Figure 1). We carried out in-silico screening of a 10 million compound drug-like library. We applied two different approaches to screen the *ZINC* compound database after removing promiscuous and non drug-like compounds using PAINS filters. The first approach is based on a 3-tier docking protocol using a published crystal structure of Myc/Max bound to DNA. After defining a putative ligand-binding site as reported in the literature, the compound library was screened using the docking tool. The second approach was based on building a pharmacophore model considering of 32 compounds reported to inhibit Myc and screening the Zinc database against this pharmacophore. We obtained 69 hits from the structure-based screen and 60 hits from the ligand-based pharmacophore screen, with 32 compounds in common between the two approaches.

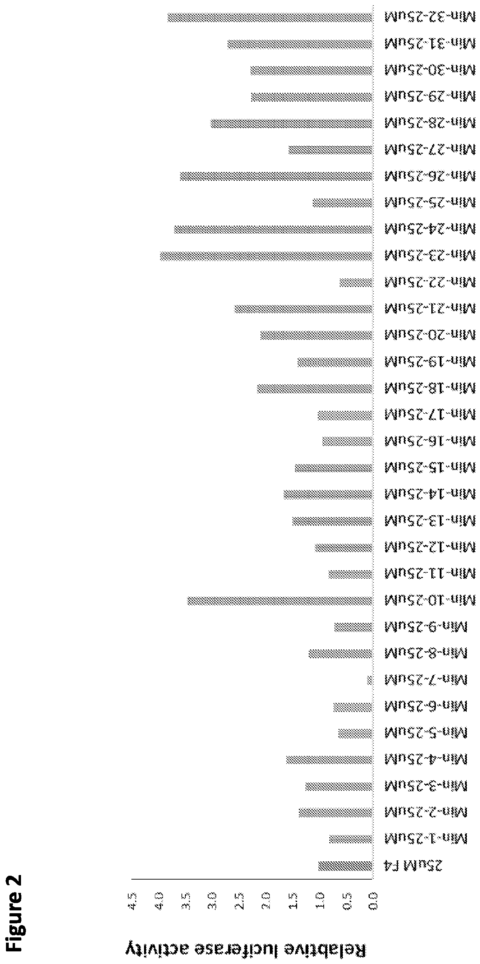

To test the compounds, we evaluated the *in silico* hits in a Myc E-Box luciferase reporter assay to measure the effects of these compounds (referred to as Min-1 to Min-32) on Myc transcriptional activity. As shown in Figure 2, about 10 compounds have similar or better activity compared to positive control 10058-F4 at 25µM. (*See* Figure 2).

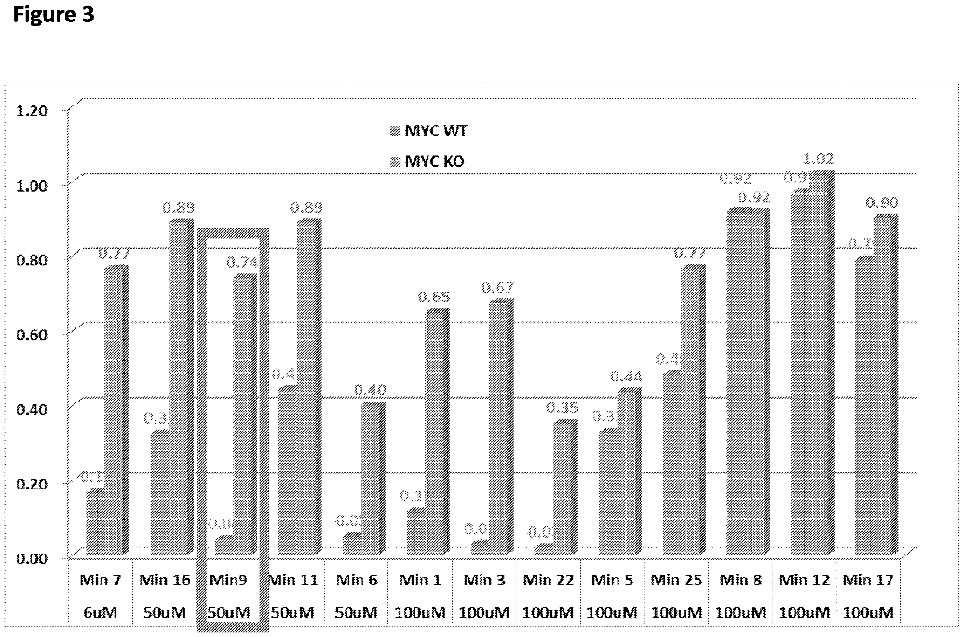

We next examined the ability of the compounds to selectively inhibit the proliferation of wild type cells expressing Myc relative to cells with Myc knockout. We tested the top 13 active compounds in the first screen assay. Figure 3 shows a graph of growth inhibition by each compound on the wild type and Myc knockout rat fibroblasts at the dose with the greatest selectivity. More than half of the tested compounds show better growth inhibitory effect on Myc WT compared to Myc KO cells. Min9-S7 (NUCC-0176248) is very promising because of its low effective concentration (6µM) and high specificity. Min9-S9 (NUCC-0176250) also shows a great selectivity at an acceptable dosage (50µM)

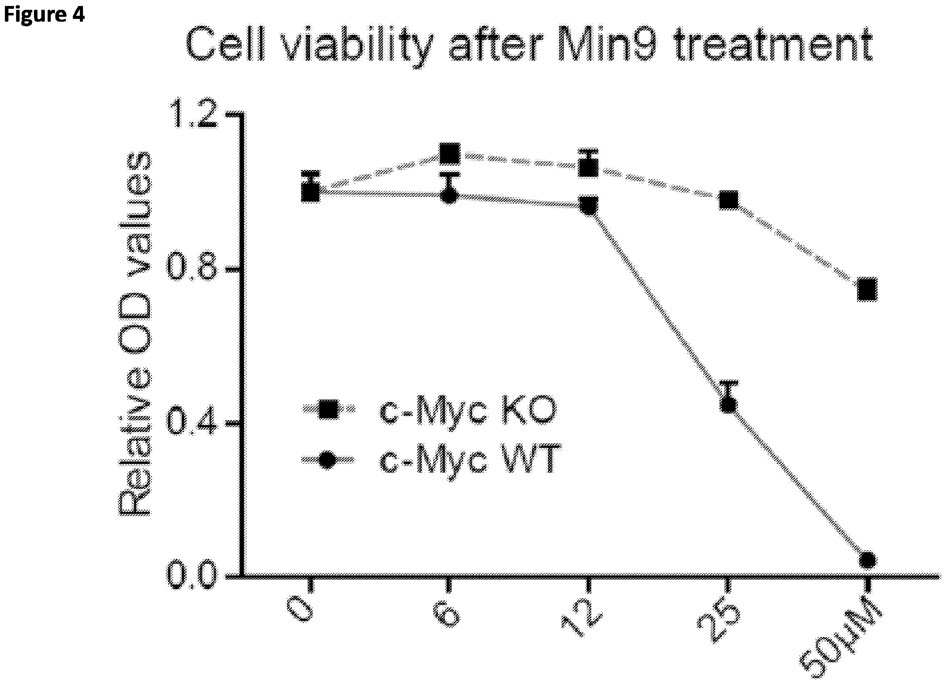

Min9 (NUCC-0176234) was also tested in a cell viability assay against a cMyc wild-type (WT) and a cMyc KO line. As shown in Figure 4, this compound reduces cell viability much more in the WT line than the KO cells, indicating a mechanism directly related to cMyc.

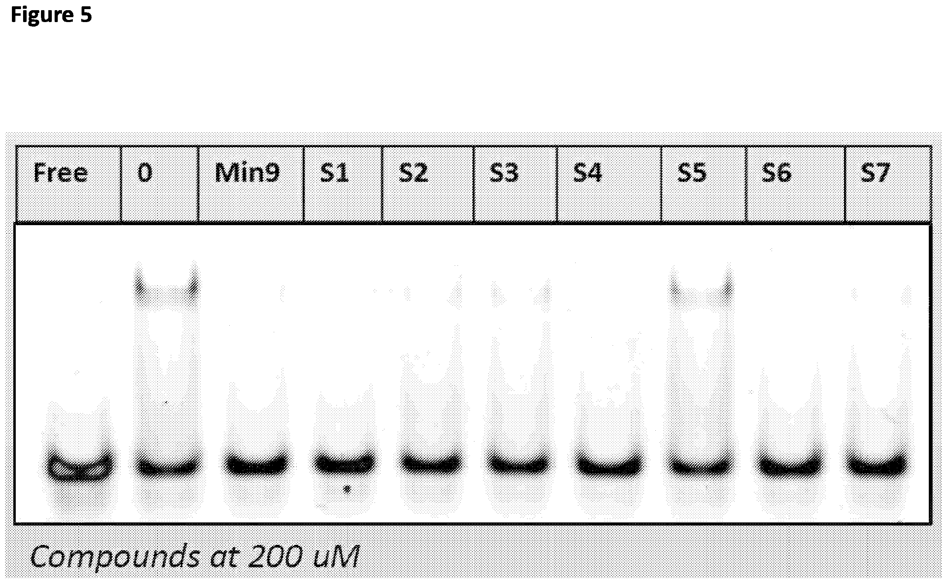

We also tested our best hit compound Min9 (NUCC-0176234) and newly synthesized analogs for effects of these compounds on Myc/Max binding to DNA in electrophoretic mobility shift assays (EMSAs). (*See* Figure 5a and Figure 5b). We expected the active compounds to impair Myc/Max binding to DNA. Several strucutural analogs of Min9 were tested over multiple doses for inhibiting Myc-DNA binding and we observed a dose-dependent inhibition. (*See* Figure 5c).

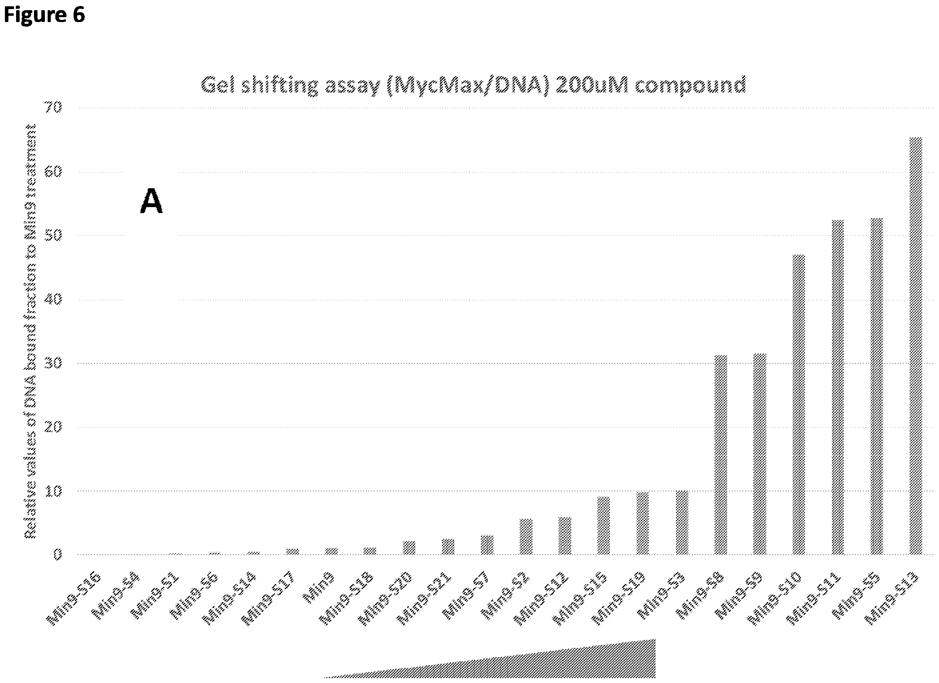

Min9 (NUCC-0176234) was also tested for its ability to cause DNA damage in an rH2AX staining assay. We would not expect cMyc-targeting agents to produce significant DNA damage. Compounds that act directly against DNA such as doxorubicin do however. We observed essentially no DNA damage caused by Min9 (NUCC-0176234). (*See* Figure 6).

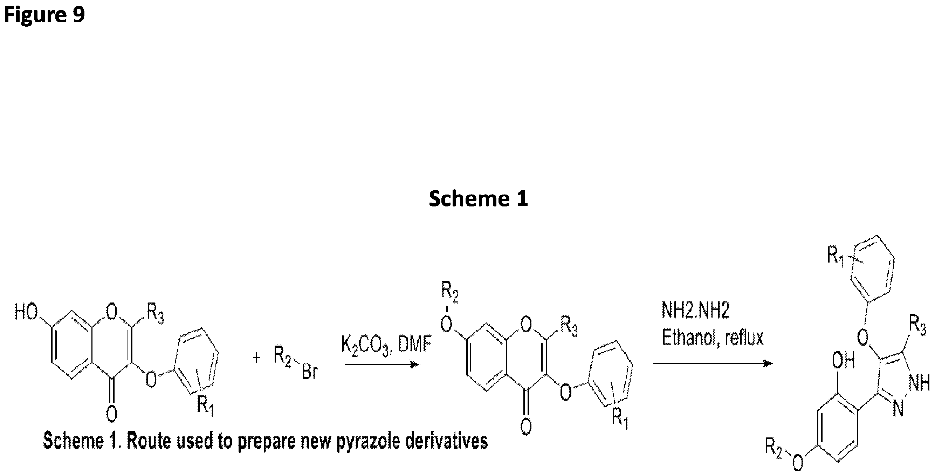

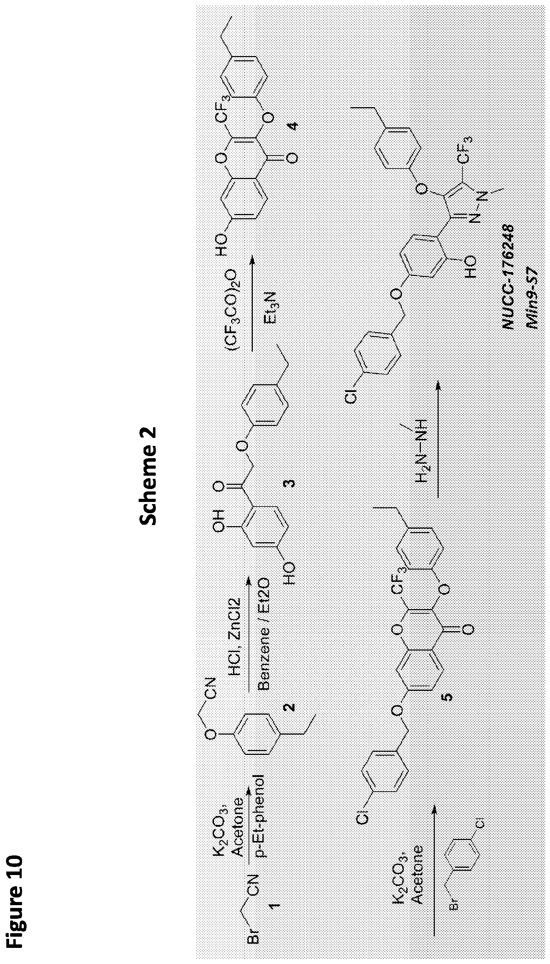

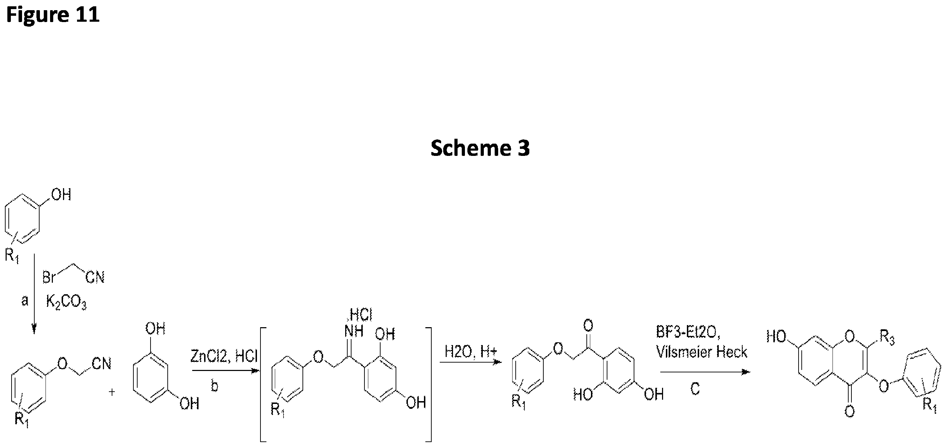

Compounds were prepared according to Scheme 1. (*See* Figure 9). Commercially available chromenones were alkylated with the desired bromide substrates using K₂CO₃ in DMF. These were then treated with hydrazine in refluxing ethanol to afford the final pyrazole derivatives. Compounds also may be prepared according to Scheme 2. (*See* Figure 10). Chromenones for Scheme 1 and Scheme 2 can be according to Scheme 3. (*See* Figure 11).

**Table 1.**

| Structure | NUCC ID | Alternate ID | % Fraction Bound |
|---|---|---|---|
| 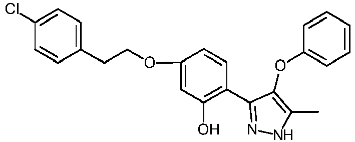 | NUCC-0176262^{∗} | Min9-S21 | 2.5 |
| 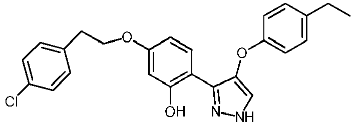 | NUCC-0176261^{∗} | Min9-S20 | 2.1 |
| 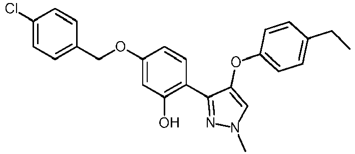 | NUCC-0176260^{∗} | Min9-S19 | 9.8 |
| 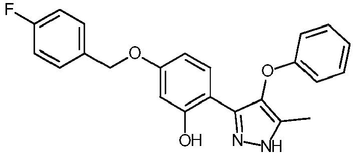 | NUCC-0176259^{∗} | Min9-S18 | 1.1 |
| 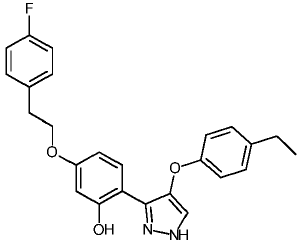 | NUCC-0176258^{∗} | Min9-S17 | 0.9 |
| 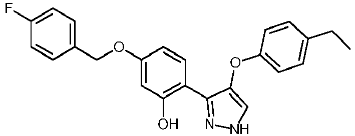 | NUCC-0176257^{∗} | Min9-S16 | 0.0 |
| 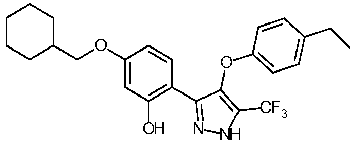 | NUCC-0176256^{∗} | Min9-S15 | 9.1 |
| 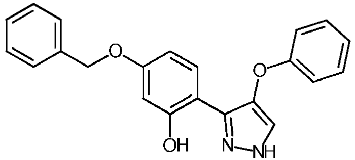 | NUCC-0176255^{∗} | Min9-S14 | 0.5 |
| 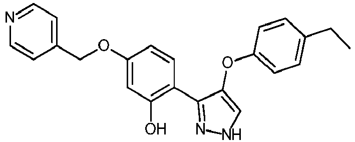 | NUCC-0176254^{∗} | Min9-S13 | 55.4 |
| 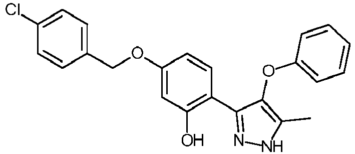 | NUCC-0176253^{∗} | Min9-S12 | 5.9 |
| 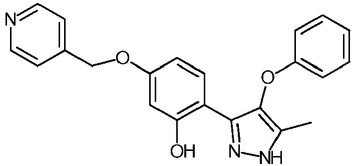 | NUCC-0176252^{∗} | Min9-S11 | 52.3 |
| 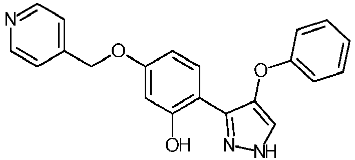 | NUCC-0176251^{∗} | Min9-S10 | 47.0 |
| 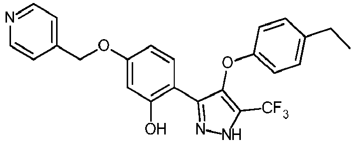 | NUCC-0176250^{∗} | Min9-S9 | 31.6 |
| 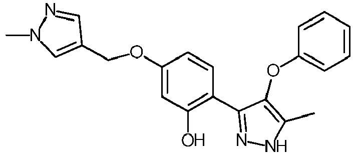 | NUCC-0176249^{∗} | Min9-S8 | 31.3 |
| 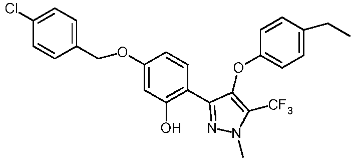 | NUCC-0176248 | Min9-S7 | 3.1 |
| 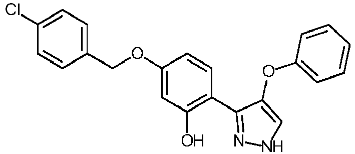 | NUCC-0176247^{∗} | Min9-S6 | 0.3 |
| 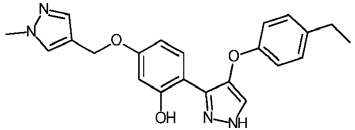 | NUCC-0176246^{∗} | Min9-S5 | 52.7 |
| 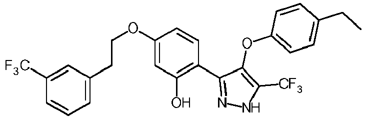 | NUCC-0176245^{∗} | Min9-S4 | 0.1 |
| 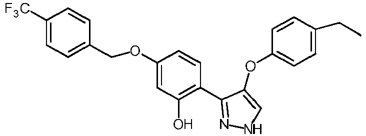 | NUCC-0176244^{∗} | Min9-S3 | 10.1 |
| 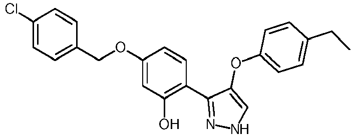 | NUCC-0176243^{∗} | Min9-S2 | 5.6 |
| 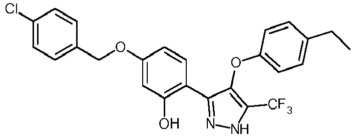 | NUCC-0176242 | Min9-S1 | 0.2 |
| 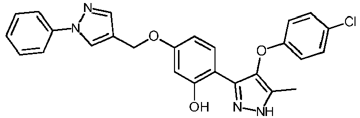 | NUCC-0176241^{∗} | Min9-5 | |
| 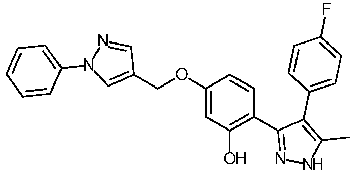 | NUCC-0176240^{∗} | Min9-3 | |
| 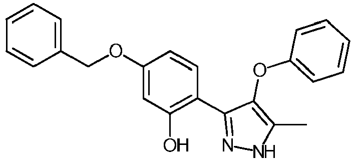 | NUCC-0176239^{∗} | Min9-7 | |
| 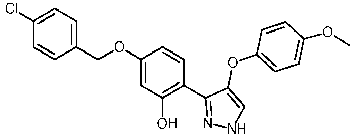 | NUCC-0176238^{∗} | Min9-8 | |
| 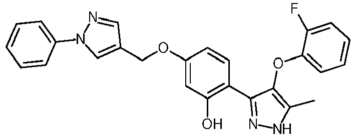 | NUCC-0176237^{∗} | Min9-2 | |
| 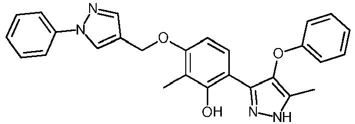 | NUCC-0176236^{∗} | Min9-6 | |
| 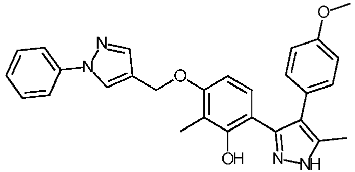 | NUCC-0176235^{∗} | Min9-1 | |
| 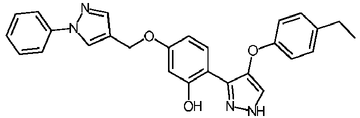 | NUCC-0176234^{∗} | Min9-0 | 1.0 |
| 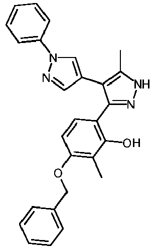 | NUCC-0020105^{∗} | Min9-4 | |

| | | | |
|---|---|---|---|
| ^{∗} indicates compounds provided for reference only. | | | |

In vitro metabolism of NUCC-176242 and NUCC-176248 were tested using mouse liver microsomes and a mouse S9 fraction. NUCC-176242 was significantly metabolism by the mouse S9 fraction versus NUCC-176248 likely due to S9 conjugation at the N-1 nitrogen atom of the pyrazole ring.

The pharmacokinetics of NUCC-176242 and NUCC-176248 were studied in mice by administering a dose of 5 mg/kg intravenously and measuring the plasma concentration versus time. The observed in vivo metabolism of NUCC-176242 and NUCC-176248 correlated well with the observed in vitro metabolism tested above for of NUCC-176242 and NUCC-176248.

### References

[1] Huang M, Weiss WA. 2013. Neuroblastoma and MYNC. Cold Spring Harb Perspect Med 3: a014415.
[2] Roussel MF, Robinson GW. 2013. Role of MYC in medulloblastoma. Cold Spring Harb Perspect Med 3: a014308.
[3] Gabay M, Li Y, Felsher DW. 2014. MYC activation is a hall mark of cancer initiation and maintenance. Cold Spring Harb Perspect Med doi: 10.1101/cshperspect.a014241.
[4] Schmitz R, Ceribelli M, et. al. 2014. Oncogenic mechanisms in Burkitt lymphoma. Cold Spring Harb Perspect Med 4: a014282.
[5] Michael R. McKeown and James E. Bradner, Cold Spring Harb Perspect Med 2014;4:a014266
[6] Soucek L, Whitfield JR, et. al. 2013. Inhibition of Myc family proteins eradicates KRas-driven lung cancer in mice. Genes Dev 27: 504-513.
[7] S. Fletcher, E.V. Prochownik, Small-molecule inhibitors of the Myc oncoprotein, Biochim. Biophys. Acta (2014).

### Example 2 - Representative synthesis of Substituted Heterocycles

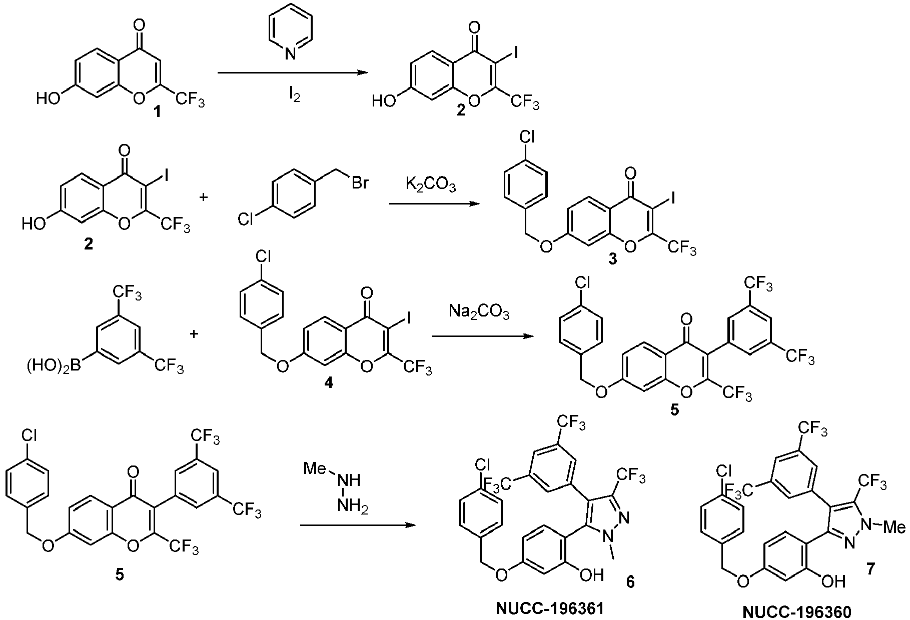

Synthesis of 7-hydroxy-3-iodo-2-(trifluoromethyl)-4H-chromen-4-one (2). In a 250mL-RBFl 7-hydroxy-2-(trifluoromethyl)-4H-chromen-4-one (1.15 g, 5.00 mmol), pyrdine (0.707 ml, 8.74 mmol), iodine (2.219 g, 8.74 mmol) and pyridine (0.707 ml, 8.74 mmol) were added. The reaction stirred overnight (15h). Reaction was quenched by addition of Na₂S₂O₃ sol, stirred for 1h. Then, it was extracted with DCM x3, dried over Na2SO4, filtrated and concentrated. It was titrated with Et2O to afford the title compound (3.44 g, 69%). MS: 357.10 (ESI+). 1H-NMR (500 MHZ, CDCl₃) δ 8.10 - 8.15 (d, J = 8.55 Hz, 1H), 7.16 (d, J = 8.54 Hz, 1H), 6.75 (s, 1H), 6.34 (br. s., 1H).

Synthesis of 7-((4-chlorobenzyl)oxy)-3-iodo-2-(trifluoromethyl)-4H-chromen-4-one (3). In a 40 ml-vial with 7-hydroxy-3-iodo-2-(trifluoromethyl)-4H-chromen-4-one (595 mg, 1.671 mmol), 1-(bromomethyl)-4-chlorobenzene (412 mg, 2.005 mmol), and K2CO3 (693 mg, 5.01 mmol) were added. Then acetone (20 ml) was added. The reaction was heated at 60 °C overnight. The LCMS as well as HNMR confirmed pure product. The reaction was quenched by adding 10 mL of 10 mL 1M HCl and extracted 3x20 ml of EtOAc, dried over Na2SO4, filtrated and concentrated. The crude was purified by Biotage SiO2 chromatography using 0-20% MeOH in DCM, affording the title compound (0.75 g, 93%). MS (ESI+) 480.99. 1H-NMR (500 MHZ, CDCl₃) δ 8.16 (d, J = 8.85 Hz), 7.45 (d, J = 8.25 Hz, 2Hz), 7.40 (d, J = 8.25 Hz, 2H), 7.01 (d, J = 9.16 Hz, 1H), 6.71 (s, 1H), 5.29 (s, 2H).

Synthesis of 3-(3,5-bis(trifluoromethyl)phenyl)-7-((4-chlorobenzyl)oxy)-2-(trifluoromethyl)-4H-chromen-4-one (5). In a 8-mL vial, 7-((4-chlorobenzyl)oxy)-3-iodo-2-(trifluoromethyl)-4H-chromen-4-one (68 mg, 0.141 mmol), (3,5-bis(trifluoromethyl)phenyl)boronic acid (36.5 mg, 0.141 mmol), Pd(Ph3P)4 (12.26 mg, 10.61 µmol) and sodium carbonate (30.0 mg, 0.283 mmol) were added. Then, toluene (0.500 ml), ethanol (0.1 ml) and water (0.200 ml) were added. The reaction stirred at 80 °C overnight. LCMS showed the product. Crude was passed directly through a pad of SiO2 using Hex:EtOAc 1:1. TLC showed a spot with high Rf (0.9 in Hex:EtOAc (1:1). Purified by Biotage SiO₂ chromatography, using EtOAc/Hexanes to afford the title compound (69 mg, 86%). MS (ESI+) 567.08.

Synthesis of 2-(4-(3,5-bis(trifluoromethyl)phenyl)-1-methyl-3-(trifluoromethyl)-1H-pyrazol-5-yl)-5-((4-chlorobenzyl)oxy)phenol (6). Into a 20 ml-vial with 3-(3,5-bis(trifluoromethyl)phenyl)-7-((4-chlorobenzyl)oxy)-2-(trifluoromethyl)-4H-chromen-4-one (460 mg, 0.812 mmol), Ethanol (Volume: 8 ml) and methylhydrazine (0.130 ml, 2.435 mmol) were added. Reaction was stirred for 1h at 80 °C. LCMS showed 2 isomers. The crude was concentrated in the rotovap. The crude was purified by Biotage SiO2 chromatography using 0-10% MeOH/DCM to afford a mixture of the isomers 6 and 7. Isomers were separated using reverse phase preparative HPLC to afford compound 6 (20%) and 7 (40%). Compound 6. MS (ESI+) 595.13. 1H NMR (500 MHZ, CDCl₃) δ: 7.94 (s, 2H), 7.90 (s, 1H), 7.31 (d, J = 8.55 Hz, 2H), 7.25 (d, J = 8.55 Hz, 1H), 7.15 - 7.18 (m, 2H), 6.79 (d, J = 8.85 Hz, 1H), 6.62 (s, 1H), 5.08 (s, 2H), 3.87 (s, 3H). Compound 7. MS (ESI+) 595.13. 1H NMR (500 MHZ, CDCl₃) δ: 10.70 - 10.71 (s, 1H), 7.99 (s, 2H), 7.84 (s, 1H), 7.55 (d, J = 8.85 Hz, 1H), 7.29 (d, J = 8.55 Hz, 2H), 7.17 (d, J = 8.55 Hz, 2H), 6.95 (s, 1H), 6.69 (d, J = 8.55 Hz, 1H), 5.05 (s, 2H), 4.03 (s, 3H).

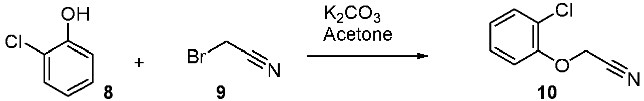

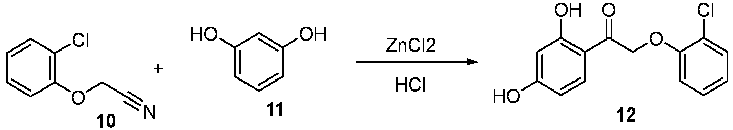

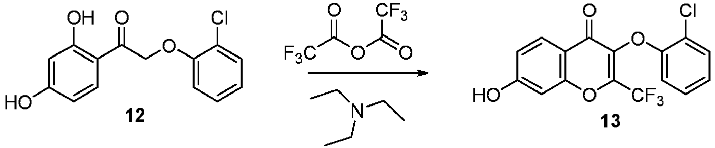

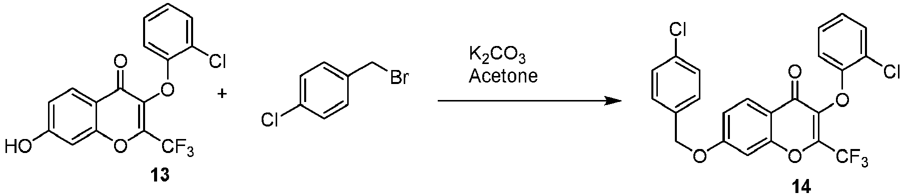

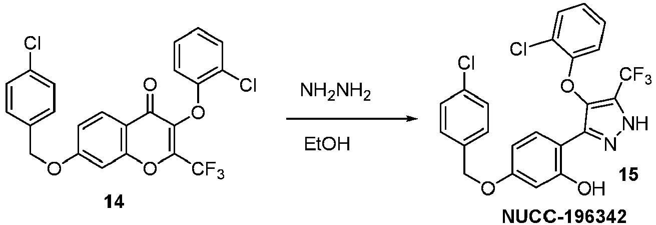

Synthesis of 2-(2-chlorophenoxy)acetonitrile (10). Into a 40 mL-vial were added 2-chlorophenol (1.54 g, 11.98 mmol), acetone (24 ml), potassium carbonate (2.483 g, 17.97 mmol) and 2-bromoacetonitrile (0.918 ml, 13.18 mmol). Then the solution was stirred at 60 °C for 4 and RT for 18 hrs. Then, it was quenched by adding 25mL of NaHCO3 aq. sat. sol. and 25 of water. The suspension was extracted with 3x40 mL of EtOAc. Combined organic layers were washed with brine, dried over Na2SO4, filtrated and concentrated. The product was purified via Biotage SiO2 chromatography with EtOAc/Hexnaes to afford the title compound (3.93 g. 98%). 1H-NMR (500 MHZ, CDCl₃) δ 7.44 (dd, J = 1.53, 7.93 Hz, 1H), 7.28 - 7.33 (m, 1H), 7.06 - 7.12 (m, 2H), 4.85 (s, 2H).

Synthesis of 2-(2-chlorophenoxy)-1-(2,4-dihydroxyphenyl)ethanone (12). Into a 20-ml vial were added 2-(2-chlorophenoxy)acetonitrile (1089 mg, 6.5 mmol) solution in benzene (6.5 ml) and the solution was covered with N2 and 4.0 M HCl solution in dioxane (16.25 ml, 65.0 mmol) was added. Then it was stirred 1 h in the ice-bath. Then, a solution of resorcinol (716 mg, 6.50 mmol) and zinc(II) chloride (886 mg, 6.50 mmol) in diethyl ether (9.75 ml) (sonicated to dissolve) was added slowly followed by 4M HCl in dioxane (16.25 ml, 65.0 mmol) solution. Then, the reaction stirred overnight. The crude was centrifuged and the supernatant was removed. Then, the white solid was washed with water 2x and the waters separated (by centrifugation). The remaining white solid was crystallized with iPrOH to afford the title compound (0.75 g, 41%). MS (ESI+) 279.28.

Synthesis of 3-(2-chlorophenoxy)-7-hydroxy-2-(trifluoromethyl)-4H-chromen-4-one (13). Into a 8 ml vial, 2-(2-chlorophenoxy)-1-(2,4-dihydroxyphenyl)ethanone (72 mg, 0.258 mmol), 2,2,2-trifluoroacetic anhydride (180 µl, 1.292 mmol) and Et3N (184 µl, 1.318 mmol) were added. Then the solution was stirred at 125-130 °C for 4 h. Then it was cooled down to r.t. It was extracted with 3 x EtOAc. Combined organic layers were washed with NH4Cl aq. sat. sol. and then brine, dried over Na2SO4, filtrated and concentrated. The crude was purified by SiO2 chromatography eluting with EtOAc/Hexanes to afford the title compound. ¹H NMR (500 MHZ, CDCl₃) d: 8.08 (d, J = 9.2 Hz, 1H), 7.45 (dd, J = 7.9, 1.5 Hz, 1H), 7.11 - 7.16 (m, 1H), 7.04 (td, J = 7.8, 1.2 Hz, 1H), 6.94 - 6.99 (m, 2H), 6.76 (dd, J = 8.1, 1.4 Hz, 1H).

Synthesis of 7-((4-chlorobenzyl)oxy)-3-(2-chlorophenoxy)-2-(trifluoromethyl)-4H-chromen-4-one (14). Into a 8ml-vial with 3-(2-chlorophenoxy)-7-hydroxy-2-(trifluoromethyl)-4H-chromen-4-one (62 g, 174 mmol), 1-(bromomethyl)-4-chlorobenzene (39.3 g, 191 mmol) and K2CO3 (72.1 g, 521 mmol) were added. Then Acetone (2 ml) was added. The reaction was heated at 60 °C overnight. The crude was diluted with 2 mL of H2O and 2 mL of HCl 1M, extracted with EtOAc, washed with brine, dried over Na2SO4, filtrated and concentrated. The crude was purified by SiO2 chromotagrpahy eluting with EtOAc/Hexanes to afford the title compound.

Synthesis of 5-((4-chlorobenzyl)oxy)-2-(4-(2-chlorophenoxy)-5-(trifluoromethyl)-1H-pyrazol-3-yl)phenol (15). In a 4ml vial were added 7-((4-chlorobenzyl)oxy)-3-(2-chlorophenoxy)-2-(trifluoromethyl)-4H-chromen-4-one (0.024 g, 0.05 mmol), Ethanol (1 ml) and hydrazine (0.014 ml, 0.300 mmol). It was stirred for 1 h at 85 °C. The crude was purified through with SiO2 chromatography using MeOH/DCM to afford the title compound (24 mg, 96%). MS (ESI+) 495.10. 1H NMR (500 MHZ, CDCl₃) d: 7.68 - 7.75 (m, 1H), 7.46 (dd, J = 7.9, 1.5 Hz, 1H), 7.30 - 7.37 (m, 4H), 7.06 - 7.11 (m, 1H), 7.00 (dd, J = 7.8, 1.1 Hz, 1H), 6.68 (d, J = 8.2 Hz, 1H), 6.51 (br. s., 2H), 5.00 (s, 2H).

### Example 3 - Additional Substituted Heterocycles

Additional compounds were synthesized and tested in the EMSA assay at concentrations of 200 µM, 100 µM, and 50 µM. Percentage of DNA bound at the respective concentration of test compound is indicated in Table 2.

**Table 2**

| Structure | Compound ID NUCC No. | Bound DNA (%) (200uM) | Bound DNA (%) (100uM) | Bound DNA (%) (50uM) | EMSA ICso (uM) |
|---|---|---|---|---|---|
| 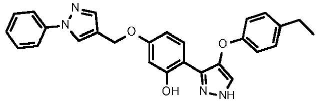 | 0176234^{∗} | 10 | 79 | | 123.1 |
| 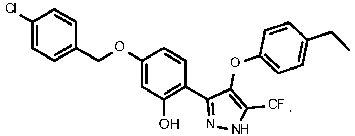 | 0176242 | 2 | 36 | | 89 |
| 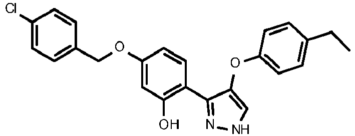 | 0176243^{∗} | 19 | | | |
| 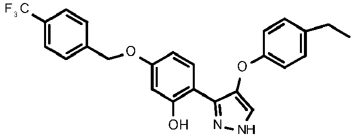 | 0176244^{∗} | 20 | | | |
| 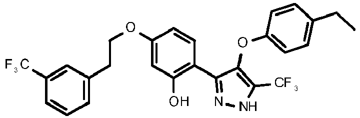 | 0176245^{∗} | 0 | 36 | | 95 |
| 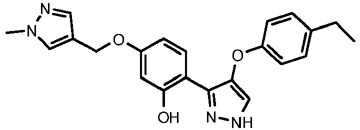 | 0176246^{∗} | 87 | | | |
| 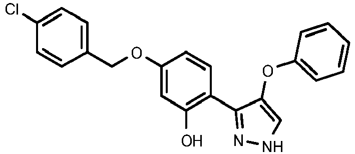 | 0176247^{∗} | 18 | | | |
| 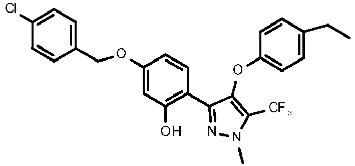 | 0176248 | 6 | 49 | | 96 |
| 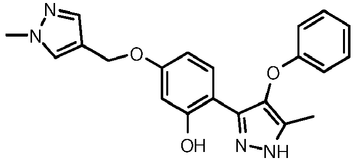 | 0176249^{∗} | 85 | | | |
| 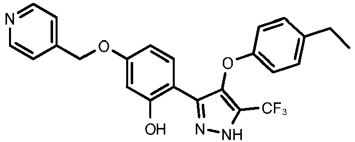 | 0176250^{∗} | 57 | | | |
| 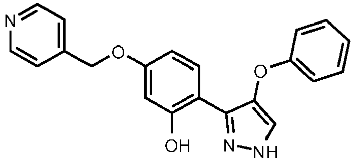 | 0176251^{∗} | 62 | | | |
| 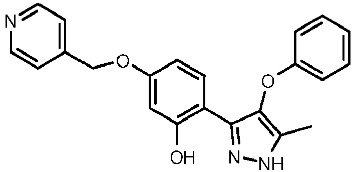 | 0176252^{∗} | 87 | | | |
| 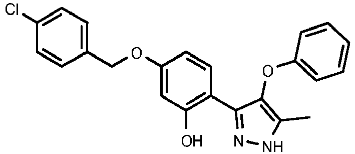 | 0176253^{∗} | 39 | | | |
| 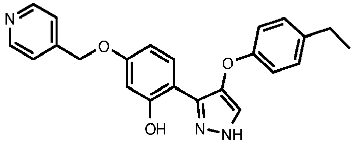 | 0176254^{∗} | 101 | | | |
| 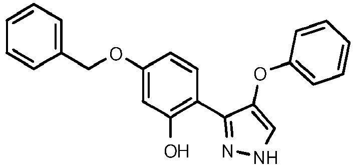 | 0176255^{∗} | 11 | | | |
| 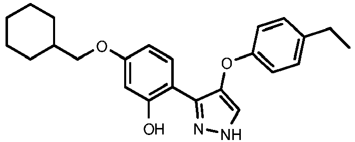 | 0176256^{∗} | 100 | | | |
| 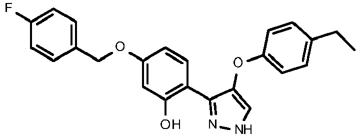 | 0176257^{∗} | 7 | | | |
| 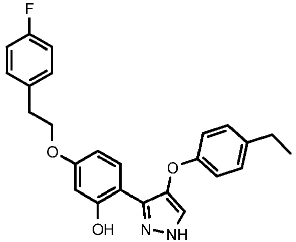 | 0176258^{∗} | 17 | | | |
| 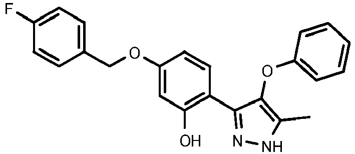 | 0176259^{∗} | 26 | | | |
| 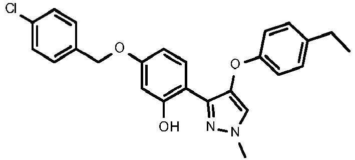 | 0176260^{∗} | 113 | | | |
| 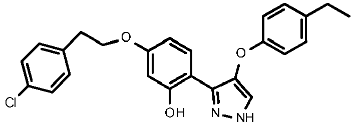 | 0176261^{∗} | 25 | | | |
| 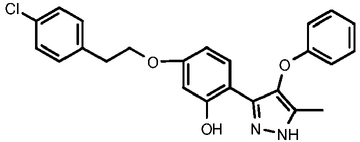 | 0176262^{∗} | 54 | | | |
| 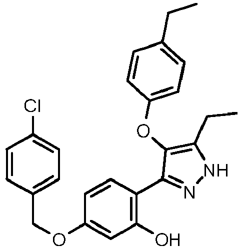 | 0196282^{∗} | | 87 | | |
| 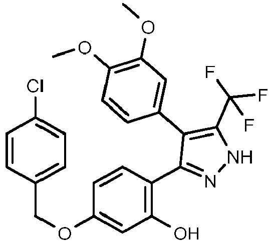 | 0196283 | | 4 | | 49 |
| 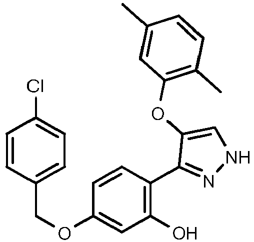 | 0196284^{∗} | | 59 | | |
| 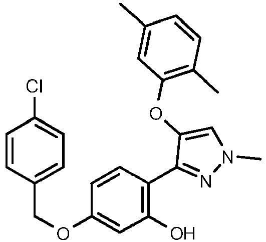 | 0196285^{∗} | | 118 | | |
| 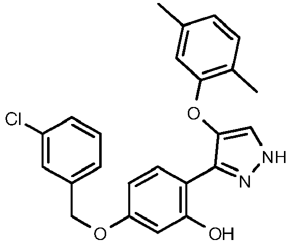 | 0196286^{∗} | | 65 | | |
| 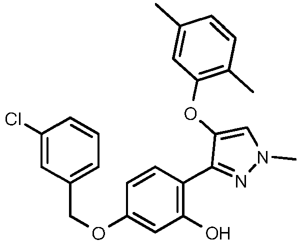 | 0196287^{∗} | | 115 | | |
| 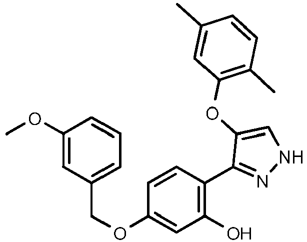 | 0196288^{∗} | | 81 | | |
| 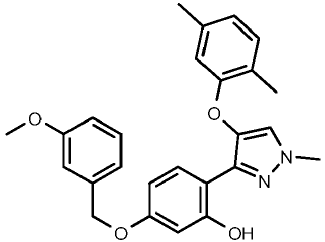 | 0196289^{∗} | | 136 | | |
| 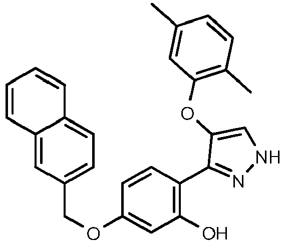 | 0196290^{∗} | | 92 | | |
| 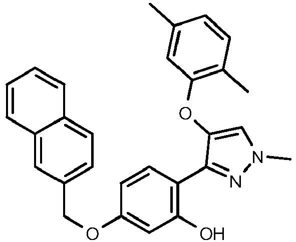 | 0196291^{∗} | | 110 | | |
| 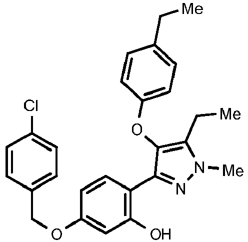 | 0196294^{∗} | | 106 | | |
| 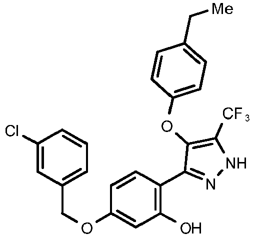 | 0196295^{∗} | | 12 | | 66 |
| 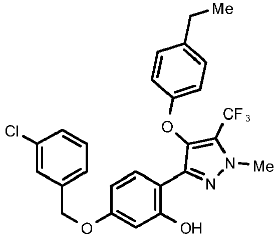 | 0196296^{∗} | | 73 | | |
| 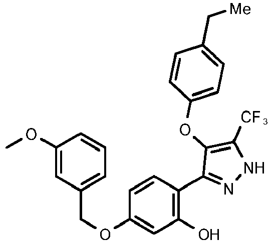 | 0196297^{∗} | | 45 | | |
| 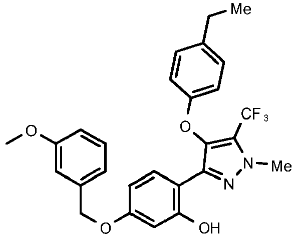 | 0196298^{∗} | | 98 | | |
| 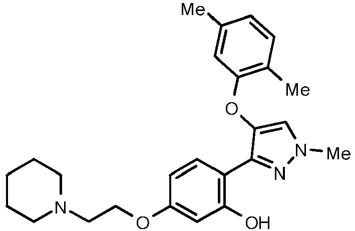 | 0196299^{∗} | | 138 | | |
| 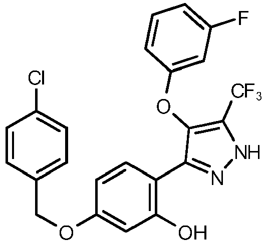 | 0196301 | | 43 | | |
| 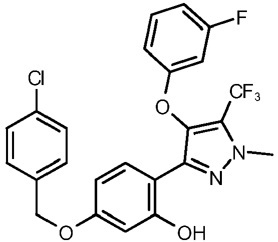 | 0196302 | | 65 | | |
| 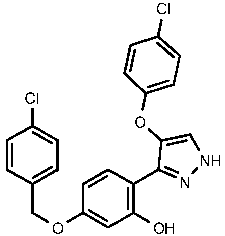 | 0196303^{∗} | | 72 | | |
| 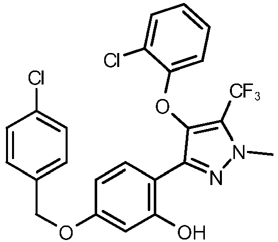 | 0196304 | | 13 | | |
| 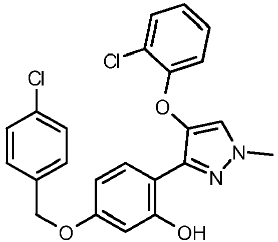 | 0196305^{∗} | | 3 | | 39 |
| 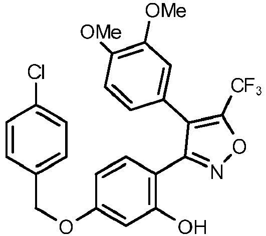 | 0196306^{∗} | | 71 | | |
| 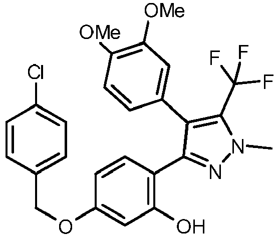 | 0196311 | | 94 | | |
| 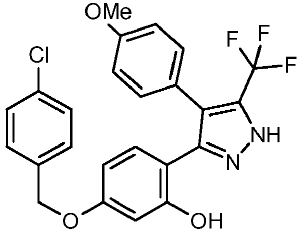 | 0196312 | | 51 | | |
| 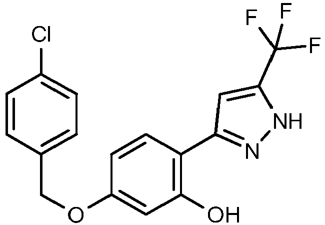 | 0196313 | | 1 | | |
| 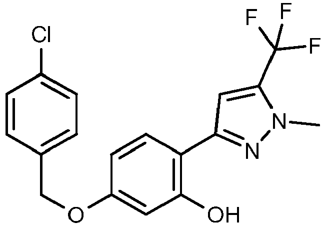 | 0196314 | | 76 | | |
| 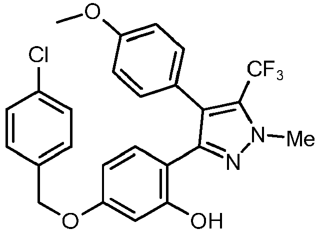 | 0196340 | | 35 | | |
| 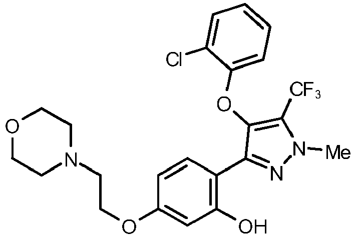 | 0196341^{∗} | | 154 | | |
| 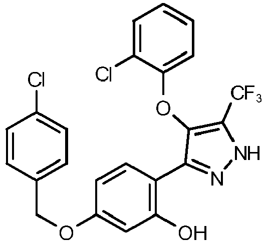 | 0196342 | | 1 | | |
| 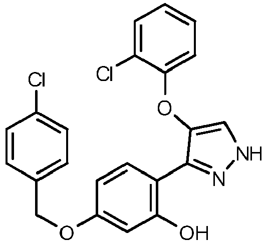 | 0196343^{∗} | | 48 | | |
| 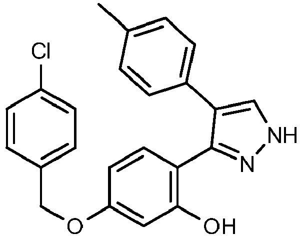 | 0196345^{∗} | | 102 | | |
| 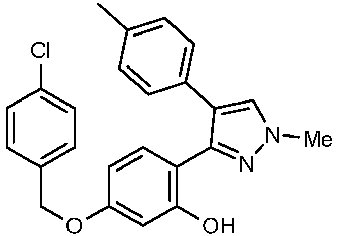 | 0196346^{∗} | | 114 | | |
| 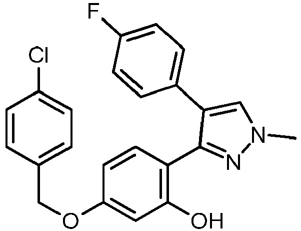 | 0196349^{∗} | | 135 | | |
| 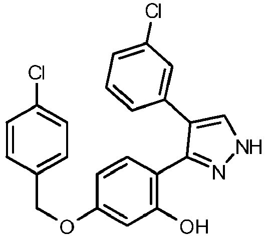 | 0196350^{∗} | | 18 | | |
| 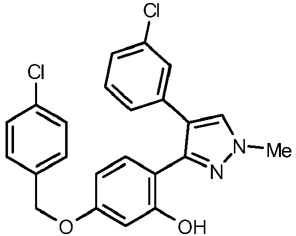 | 0196351^{∗} | | 130 | | |
| 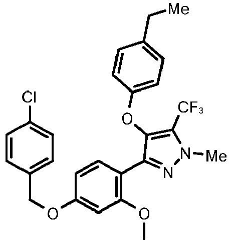 | 0196352^{∗} | | 65 | | |
| 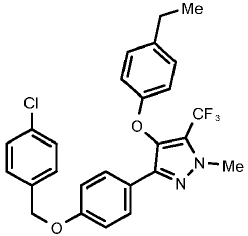 | 0196353^{∗} | | 122 | | |
| 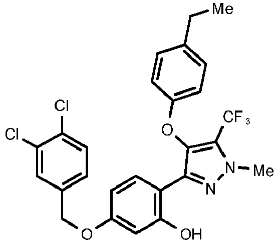 | 0196354 | | 82 | | |
| 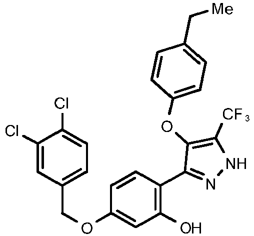 | 0196355 | | 25 | | |
| 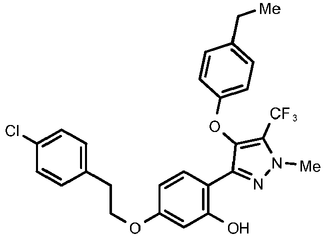 | 0196356^{∗} | | 68 | | |
| 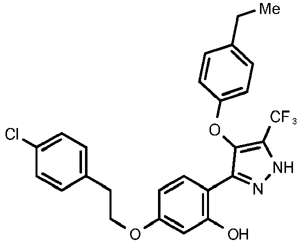 | 0196357^{∗} | | 28 | | |
| 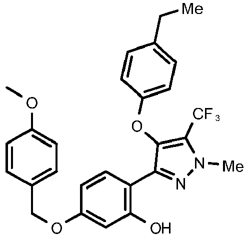 | 0196358^{∗} | | 98 | | |
| 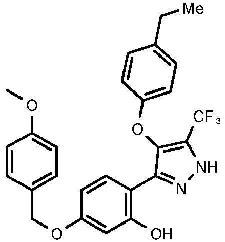 | 0196359^{∗} | | 57 | | |
| 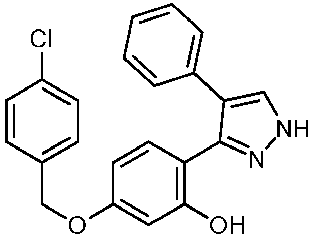 | 0198293^{∗} | | 70 | | |
| 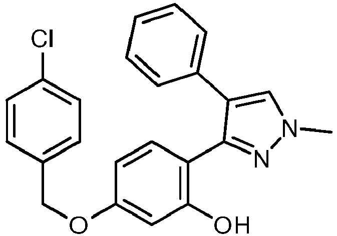 | 0198294^{∗} | | 97 | | |
| 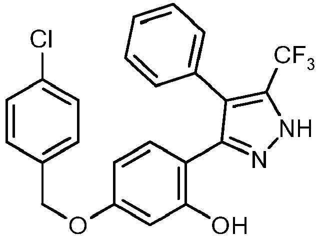 | 0198295 | | 0 | | |
| 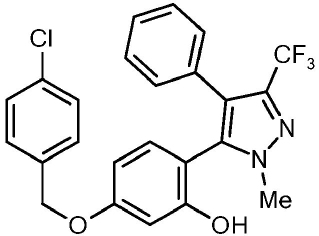 | 0198296 | | 74 | | |
| 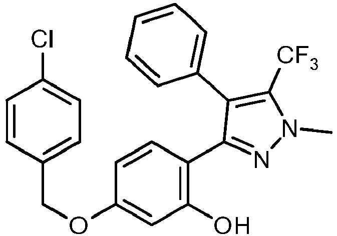 | 0198297 | | 89 | | |
| 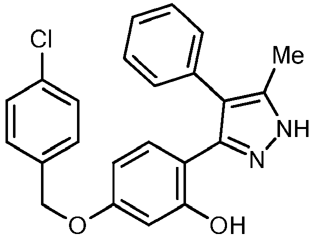 | 0198298^{∗} | | 88 | | |
| 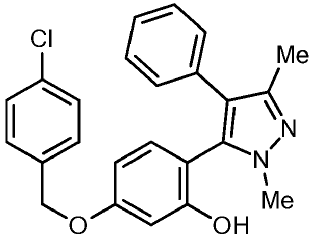 | 0198299^{∗} | | 90 | | |
| 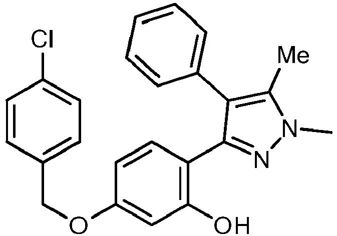 | 0198300^{∗} | | 94 | | |
| 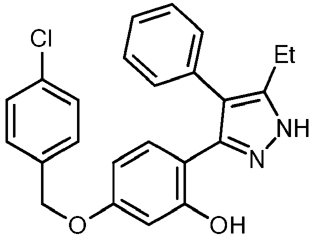 | 0198301^{∗} | | 105 | | |
| 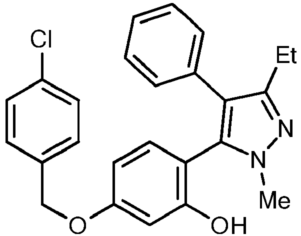 | 0198302^{∗} | | 100 | | |
| 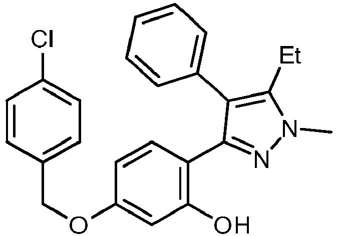 | 0198303^{∗} | | 105 | | |
| 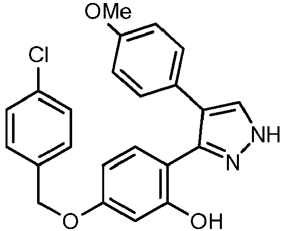 | 0198304^{∗} | | 98 | | |
| 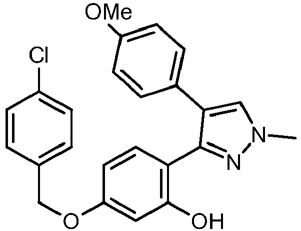 | 0198305^{∗} | | 106 | | |
| 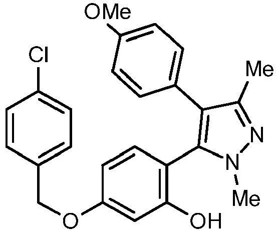 | 0198306^{∗} | | 105 | | |
| 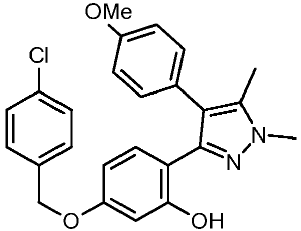 | 0198307^{∗} | | 111 | | |
| 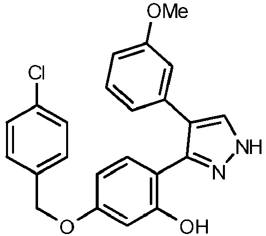 | 0198308^{∗} | | 92 | | |
| 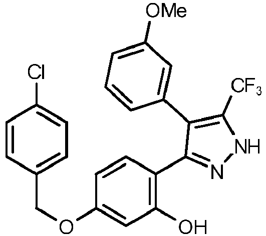 | 0198309 | | 2 | | |
| 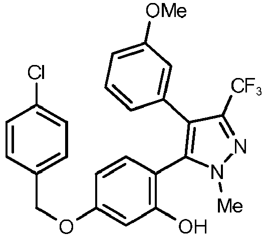 | 0198310 | | 86 | | |
| 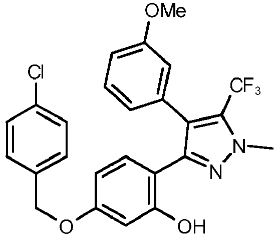 | 0198311 | | 105 | | |
| 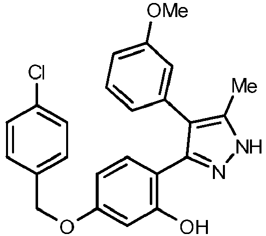 | 0198312^{∗} | | 104 | | |
| 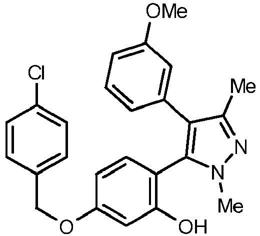 | 0198313^{∗} | | 105 | | |
| 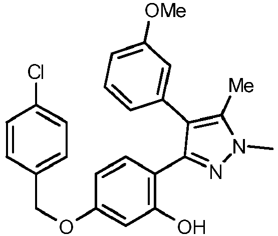 | 0198314^{∗} | | 104 | | |
| 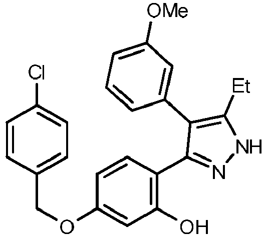 | 0198315^{∗} | | 105 | | |
| 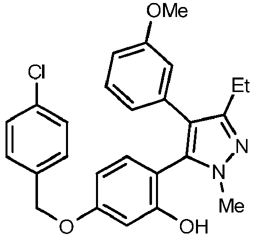 | 0198316^{∗} | | 101 | | |
| 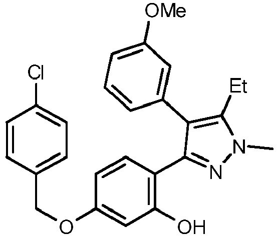 | 0198317^{∗} | | 126 | | |
| 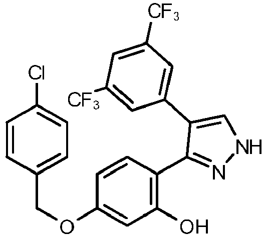 | 0198318^{∗} | | 32 | | |
| 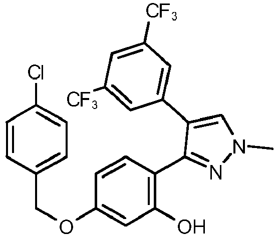 | 0198319^{∗} | | 96 | | |
| 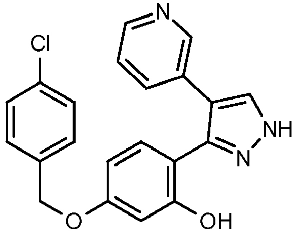 | 0198320^{∗} | | 52 | | |
| 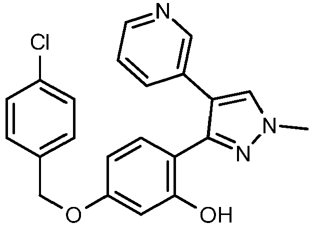 | 0198321^{∗} | | 96 | | |
| 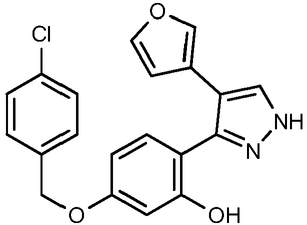 | 0198322^{∗} | | 28 | | |
| 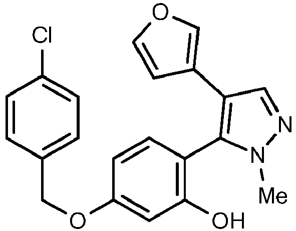 | 0198323^{∗} | | 70 | | |
| 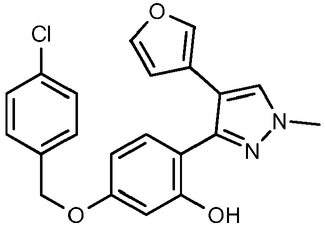 | 0198324^{∗} | | 80 | | |
| 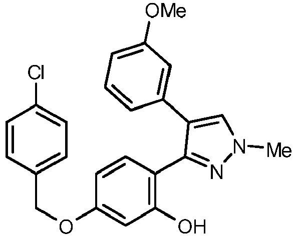 | 0198326^{∗} | | 106 | | |
| 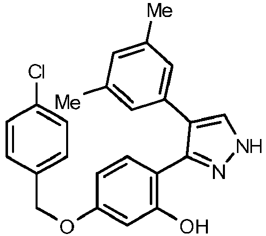 | 0198352^{∗} | | 56 | | |
| 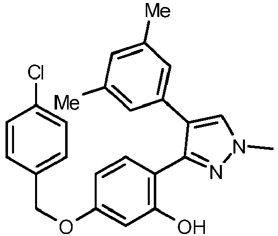 | 0198353^{∗} | | 104 | | |
| 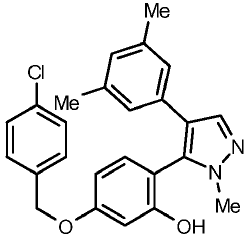 | 0198354^{∗} | | 85 | | |
| 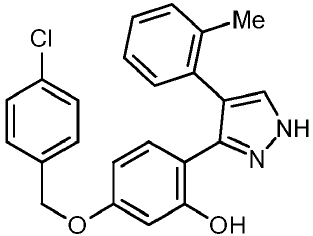 | 0198355^{∗} | | 75 | | |
| 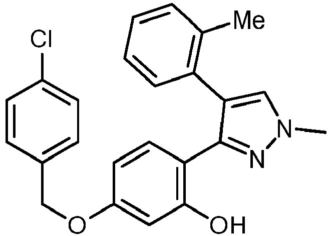 | 0198356^{∗} | | 118 | | |
| 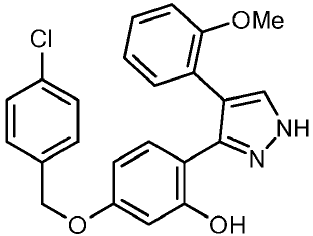 | 0198357^{∗} | | 62 | | |
| 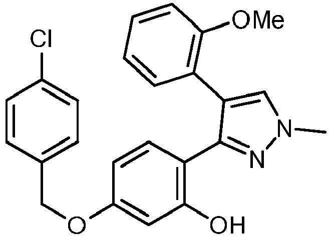 | 0198358^{∗} | | 93 | | |
| 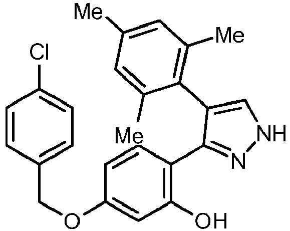 | 0198359^{∗} | | 35 | | |
| 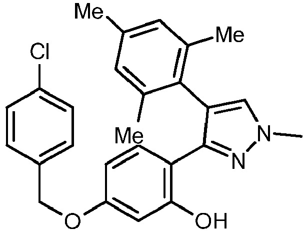 | 0198360^{∗} | | 101 | | |
| 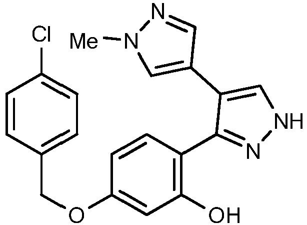 | 0198361^{∗} | | 87 | | |
| 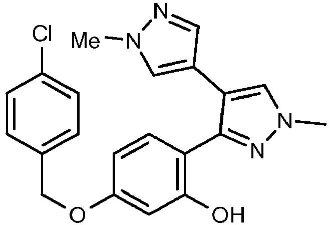 | 0198362^{∗} | | 109 | | |
| 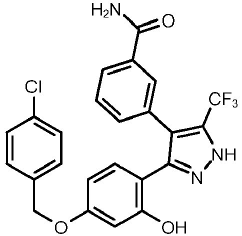 | 0198401 | | 77 | | |
| 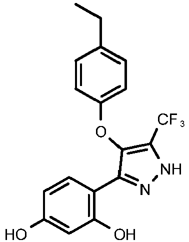 | 0198406^{∗} | | 0 | | |
| 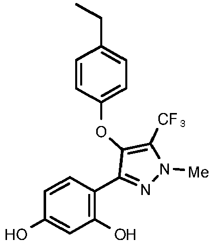 | 0198411^{∗} | | 30 | | |
| 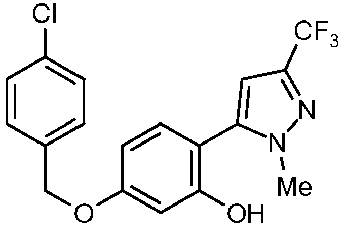 | 0198412 | | 91 | | |
| 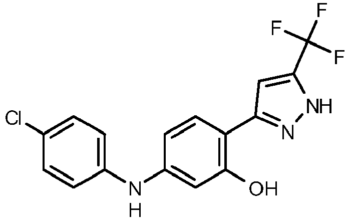 | 0200491^{∗} | | 0 | 73 | |
| 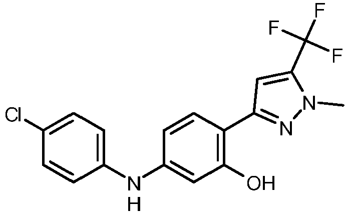 | 0200492^{∗} | | 24 | 92 | |
| 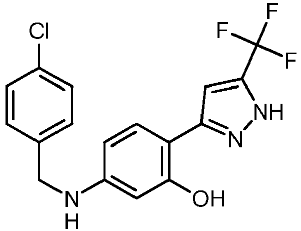 | 0200493^{∗} | | 42 | 92 | |
| 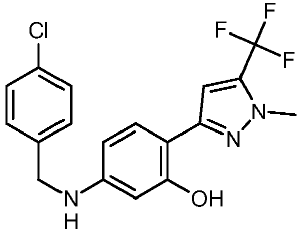 | 0200494^{∗} | | 89 | 99 | |
| 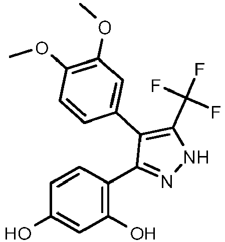 | 0200496^{∗} | | 81 | 98 | |
| 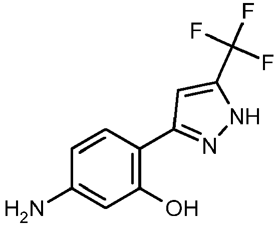 | 0200497^{∗} | | 109 | 107 | |
| 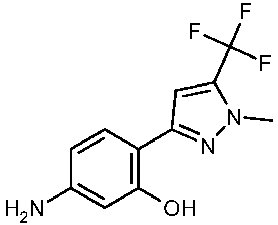 | 0200498^{∗} | | 101 | 100 | |
| 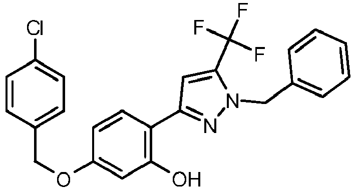 | 0200499 | | 100 | 109 | |
| 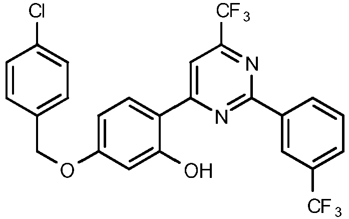 | 0200500^{∗} | | 109 | 100 | |
| 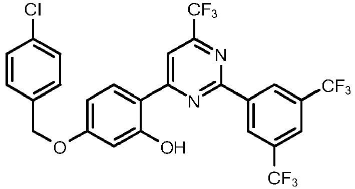 | 0200501^{∗} | | 79 | 91 | |
| 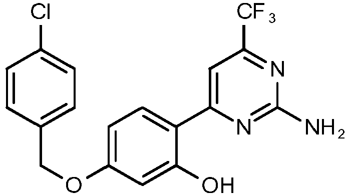 | 0200502^{∗} | | 94 | 64 | |
| 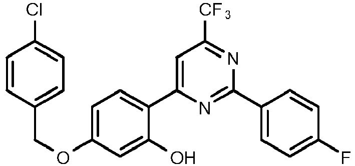 | 0200503^{∗} | | 91 | 89 | |
| 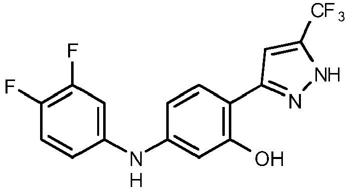 | 0200559^{∗} | | 39 | 76 | |
| 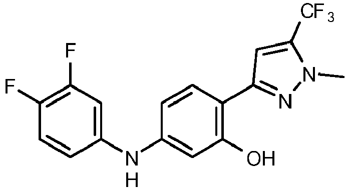 | 0200560^{∗} | | 56 | 91 | |
| 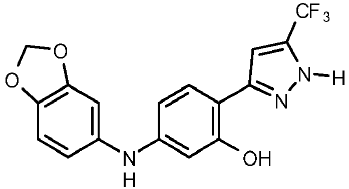 | 0200561^{∗} | | 59 | 91 | |
| 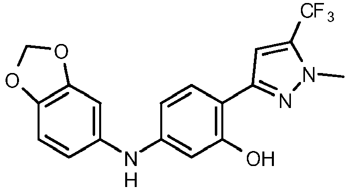 | 0200562^{∗} | | 77 | 101 | |
| 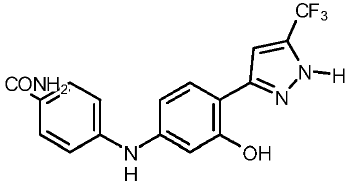 | 0200563^{∗} | | 92 | 101 | |
| 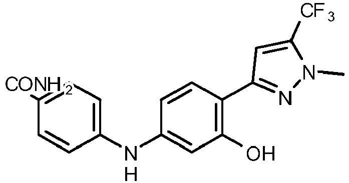 | 0200564^{∗} | | 93 | 103 | |
| 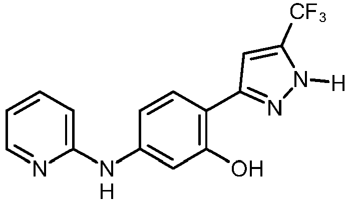 | 0200565^{∗} | | 112 | 112 | |
| 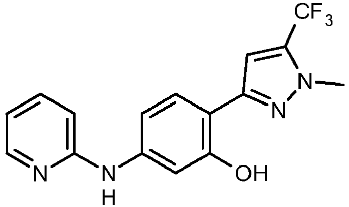 | 0200566^{∗} | | 118 | 109 | |
| 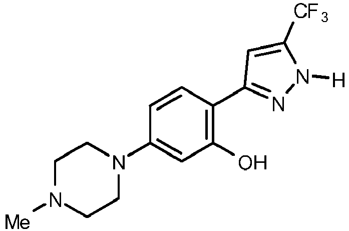 | 0200567^{∗} | | 115 | 117 | |
| 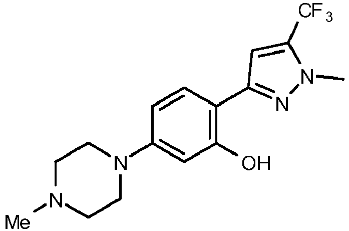 | 0200568^{∗} | | 93 | 109 | |
| 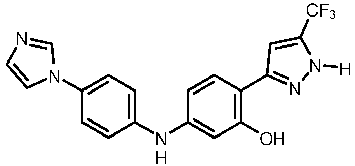 | 0200569^{∗} | | 91 | 89 | |
| 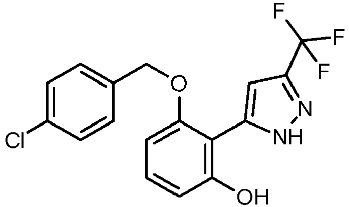 | 0200570^{∗} | | 83 | 85 | |
| 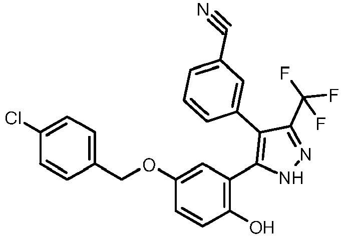 | 0200571^{∗} | | 72 | 87 | |
| 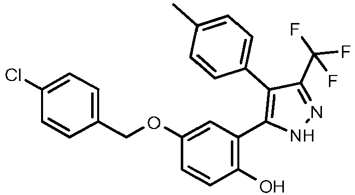 | 0200572^{∗} | | 78 | 85 | |
| 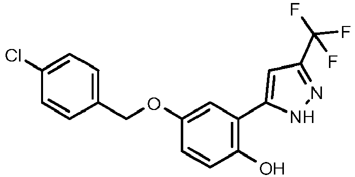 | 0200573^{∗} | | 102 | | |
| 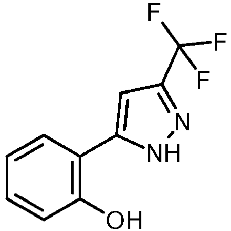 | 0200574^{∗} | | 107 | | |
| 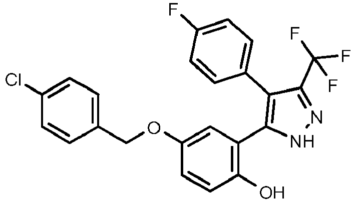 | 0200575^{∗} | | 13 | | |
| 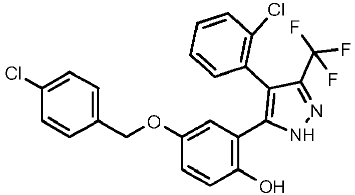 | 0200576^{∗} | | 3 | | |
| 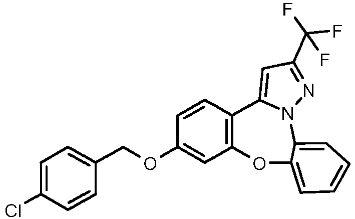 | 0200577^{∗} | | 100 | | |
| 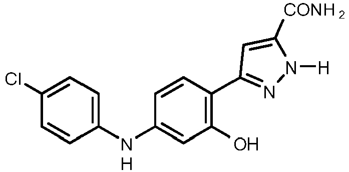 | 0200677^{∗} | | 102 | | |
| 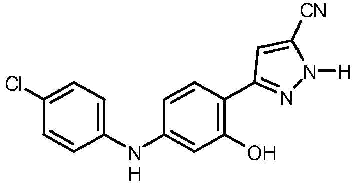 | 0200678^{∗} | | 104 | | |
| 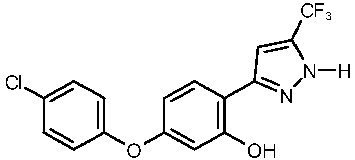 | 0200679^{∗} | | 38 | 79 | |
| 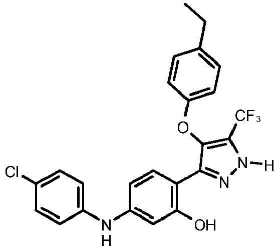 | 0200680^{∗} | | 40 | 96 | |
| 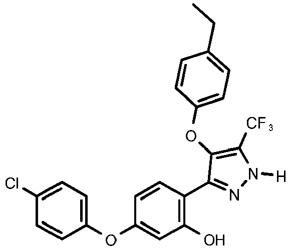 | 0200681^{∗} | | 8 | 90 | |
| 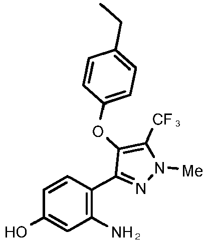 | 0200682^{∗} | | 95 | 101 | |
| 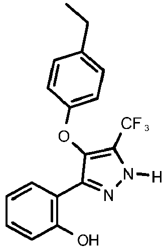 | 0200683^{∗} | | 20 | 87 | |
| 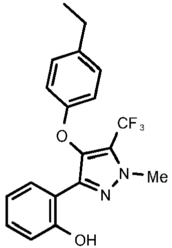 | 0200684^{∗} | | 101 | 102 | |
| 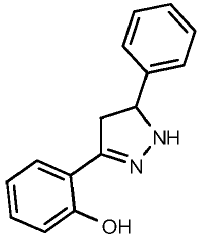 | 0200685^{∗} | | 103 | 102 | |
| 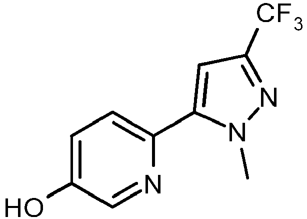 | 0200687^{∗} | | 101 | 100 | |
| 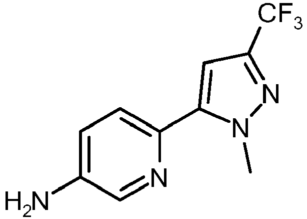 | 0200688^{∗} | | 103 | 102 | |
| 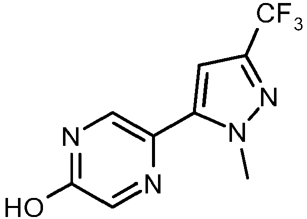 | 0200689^{∗} | | 98 | 98 | |
| 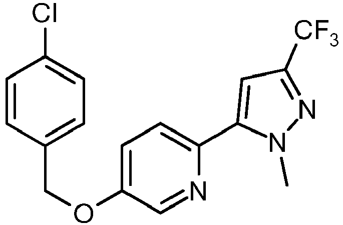 | 0200690^{∗} | | 95 | 98 | |
| 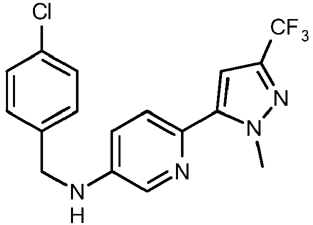 | 0200691^{∗} | | 97 | 94 | |
| 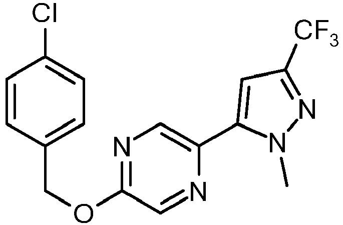 | 0200692^{∗} | | 89 | 97 | |
| 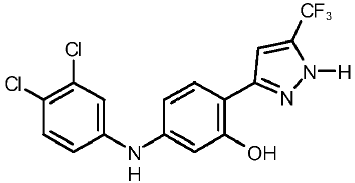 | 0200721^{∗} | | 4 | 87 | |
| 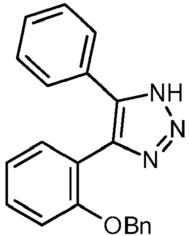 | 0200722^{∗} | | 104 | | |
| 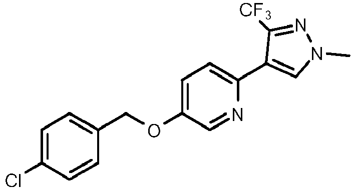 | 0200741^{∗} | | 103 | | |
| 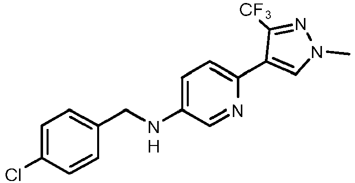 | 0200742^{∗} | | 99 | | |
| 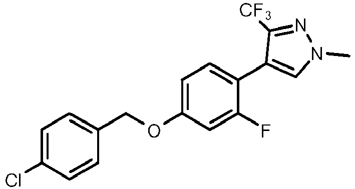 | 0200743^{∗} | | 101 | | |
| 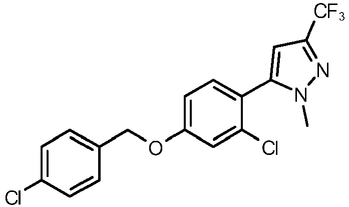 | 0200744^{∗} | | 62 | | |
| 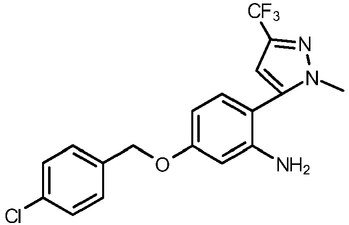 | 0200745^{∗} | | 105 | | |
| 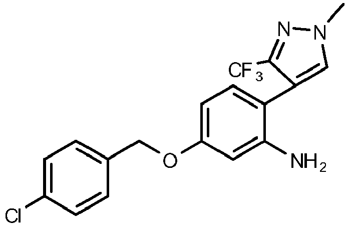 | 0200746^{∗} | | 101 | | |
| 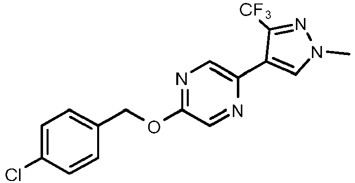 | 0200747^{∗} | | 105 | | |

| | | | | | |
|---|---|---|---|---|---|
| ^{∗} indicates compounds provided for reference only. | | | | | |

## Claims

1. A compound having a formula selected from: 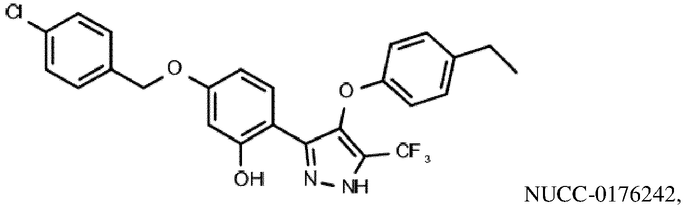 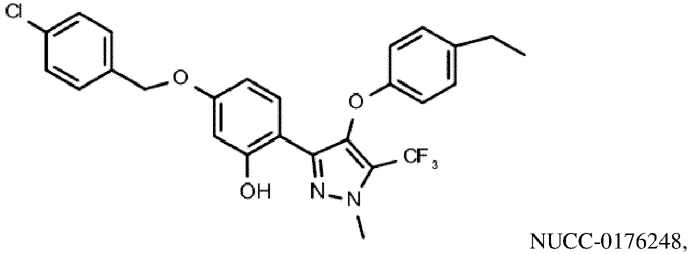 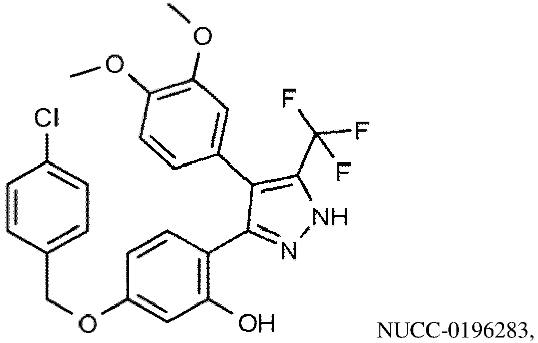 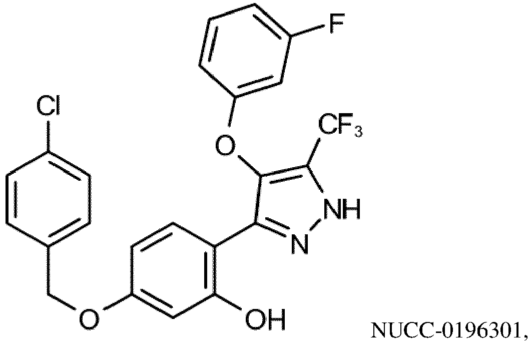 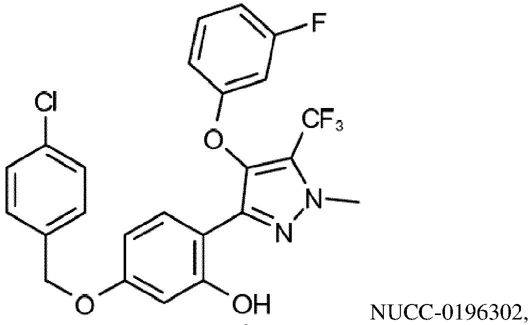 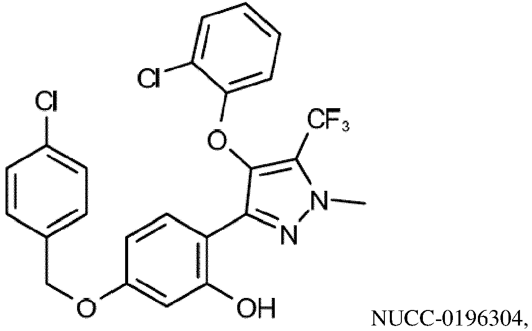 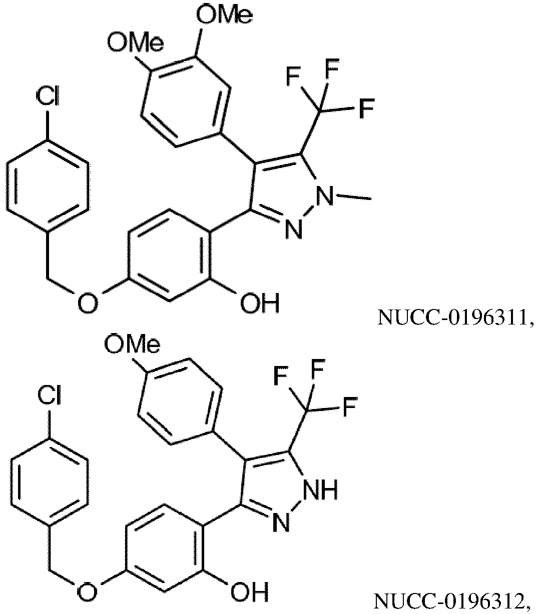 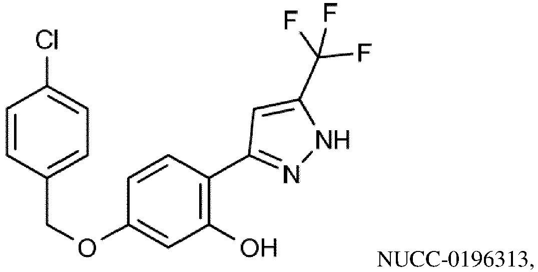 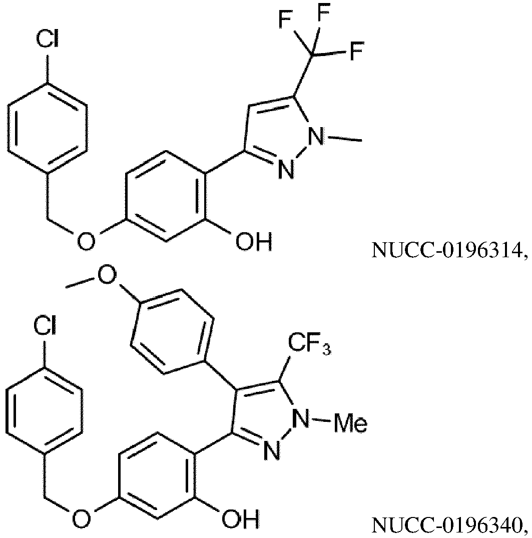 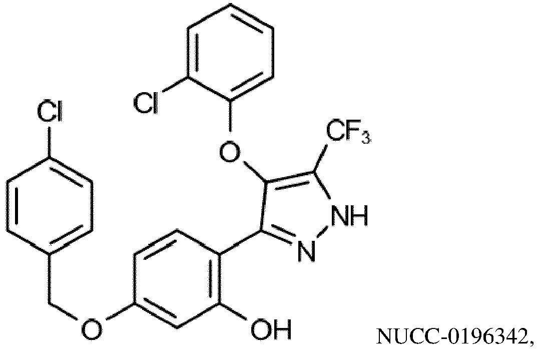 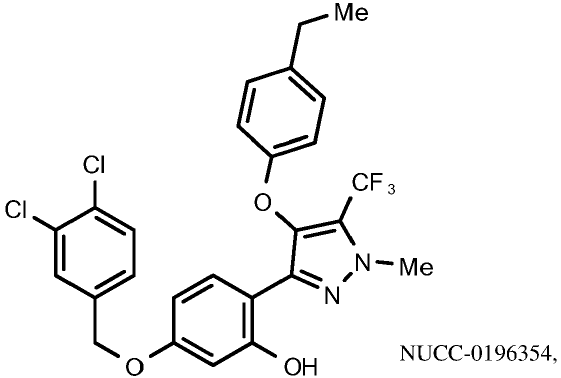 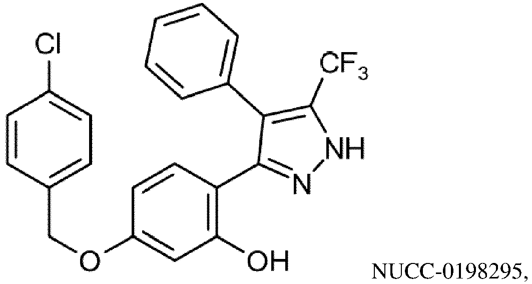 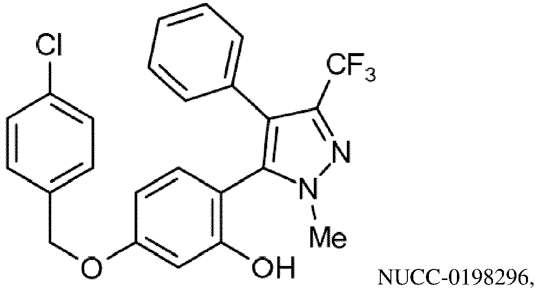 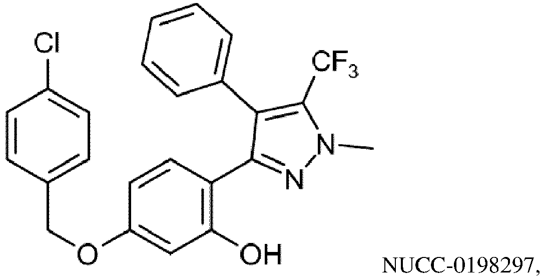 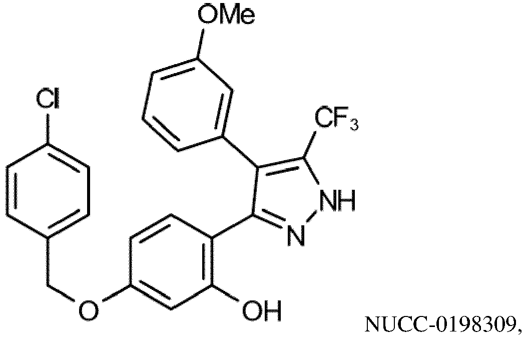 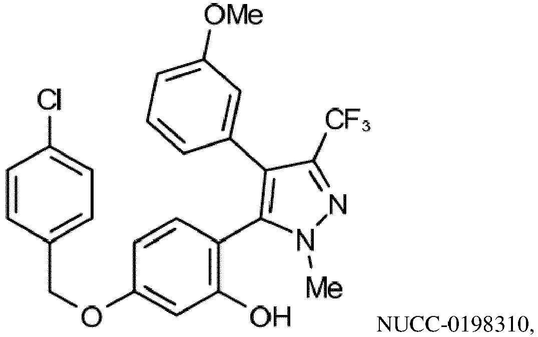 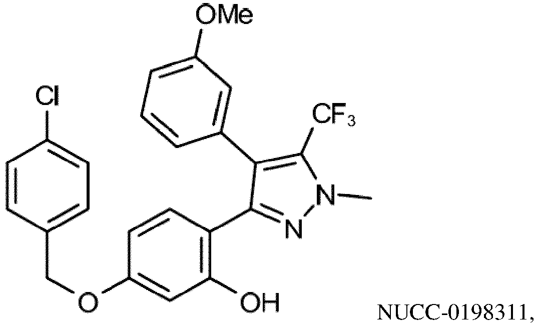 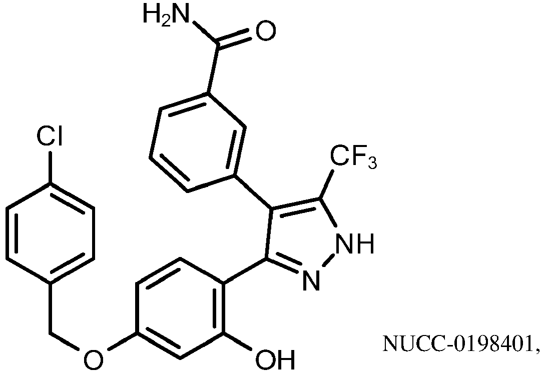 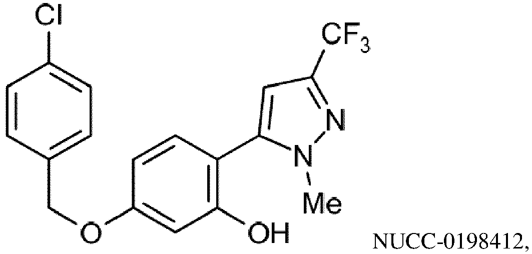 and

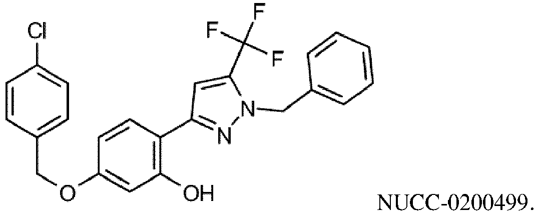

2. A pharmaceutical composition comprising a compound according to claim 1 and a suitable pharmaceutical carrier, excipient, or diluent.

3. The pharmaceutical composition of claim 2 for use in a method of treating cancer.

## Patentansprüche

1. Verbindung mit einer Formel, ausgewählt aus 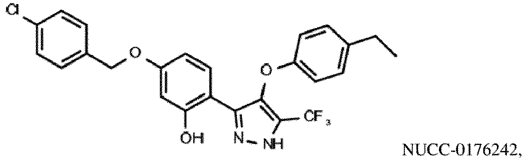 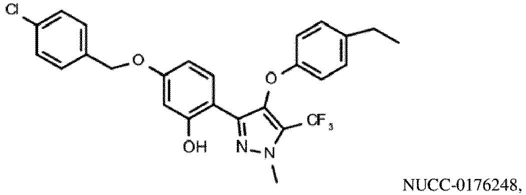 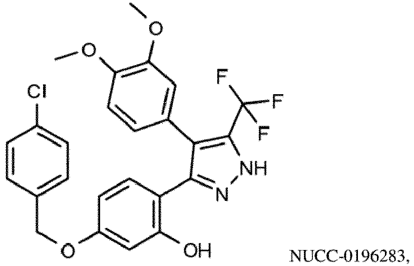 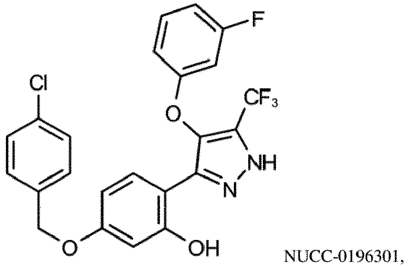 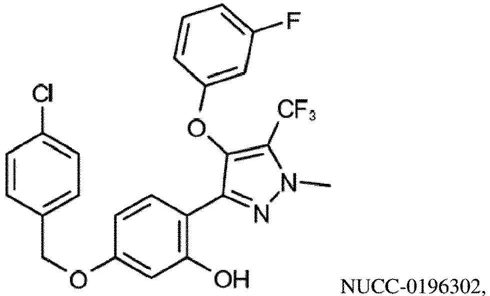 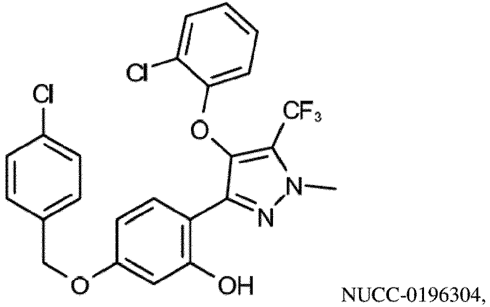 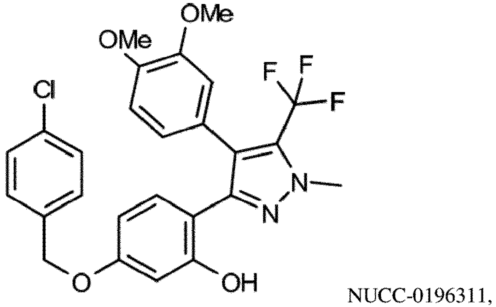 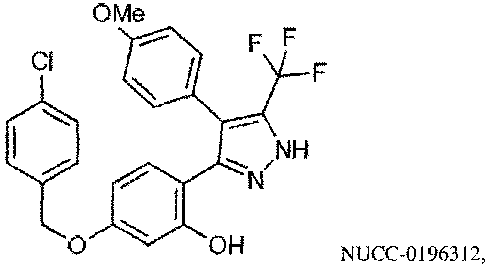 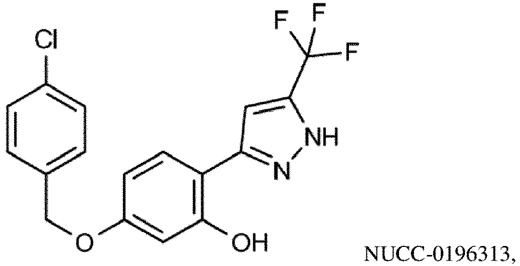 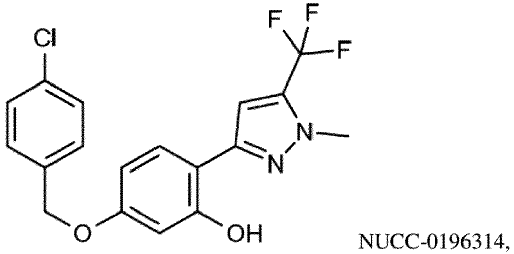 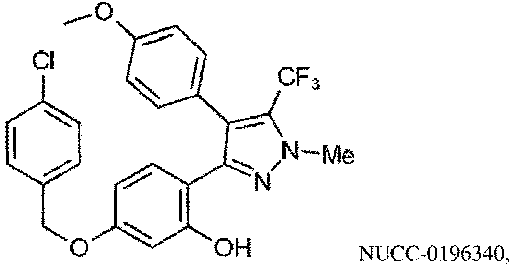 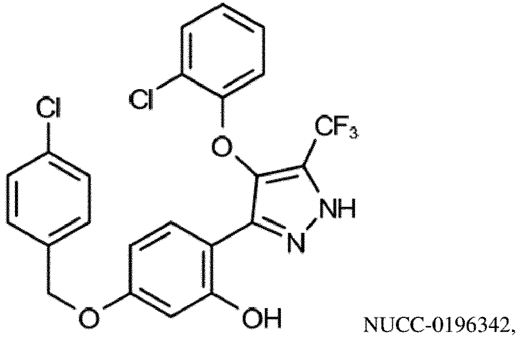 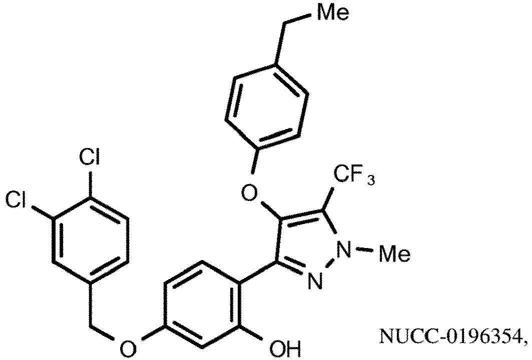 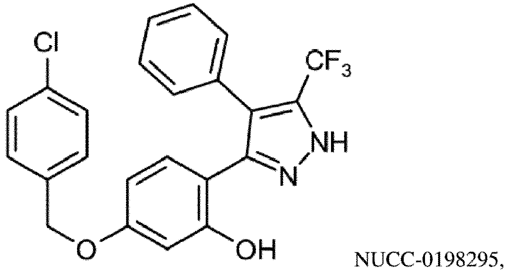 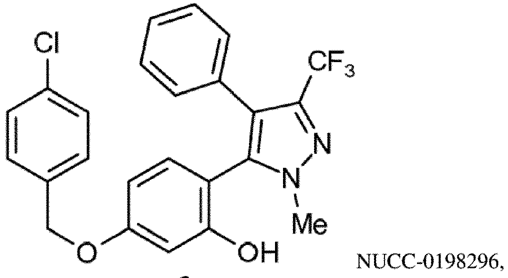 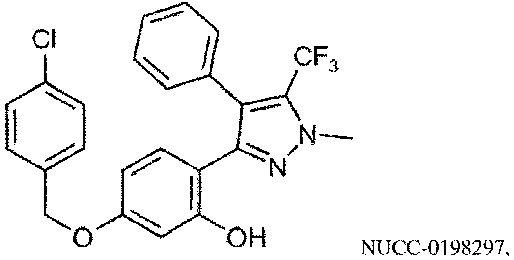 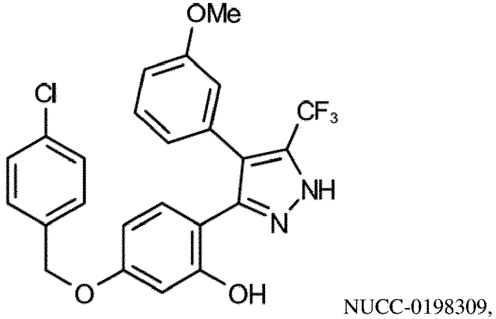 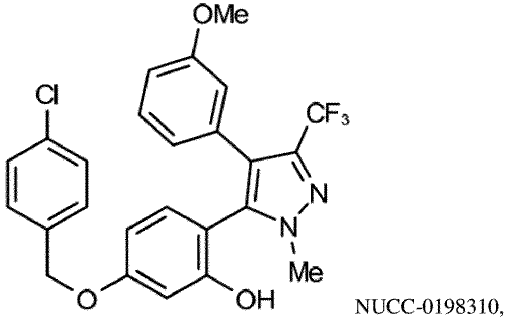 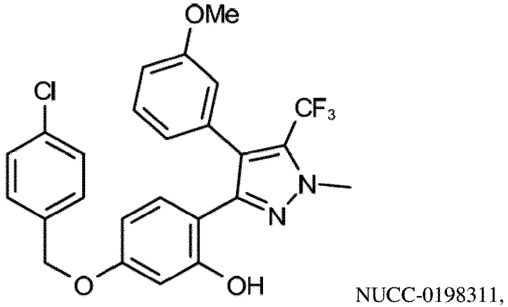 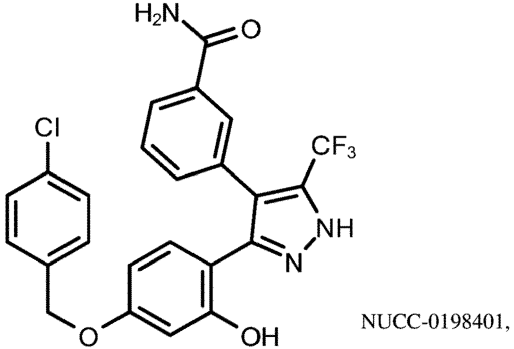 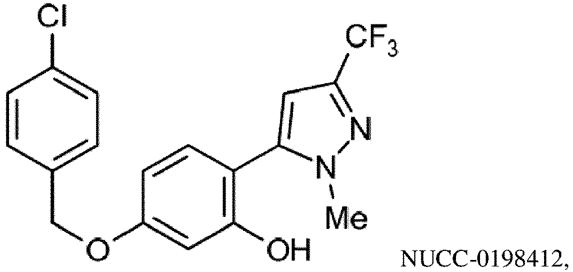 und

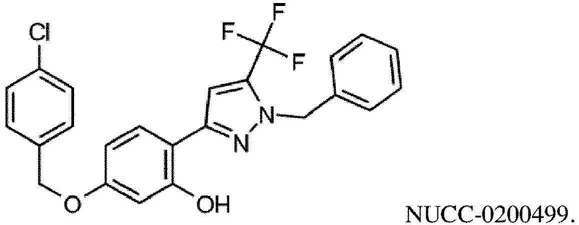

2. Pharmazeutische Zusammensetzung, umfassend eine Verbindung nach Anspruch 1 und einen geeigneten pharmazeutischen Träger, Hilfsstoff oder Verdünnungsmittel.

3. Pharmazeutische Zusammensetzung nach Anspruch 2 zur Verwendung in einem Verfahren zur Behandlung von Krebs.

## Revendications

1. Composé ayant une formule choisie parmi : 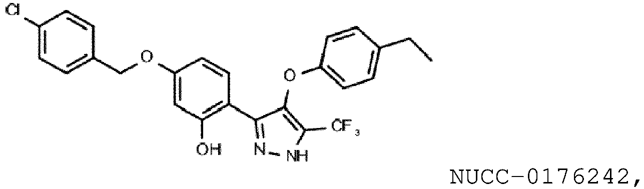 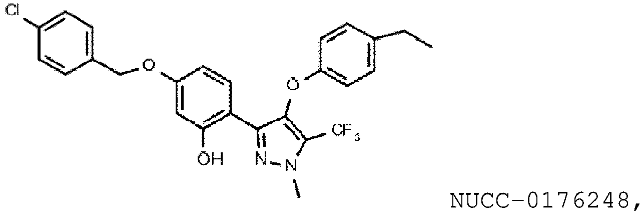 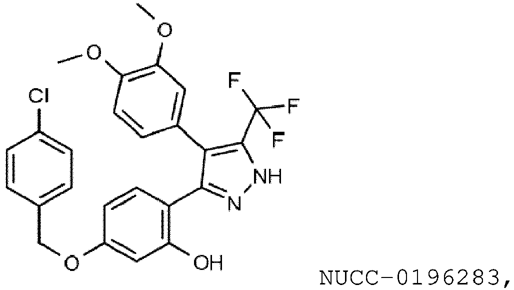 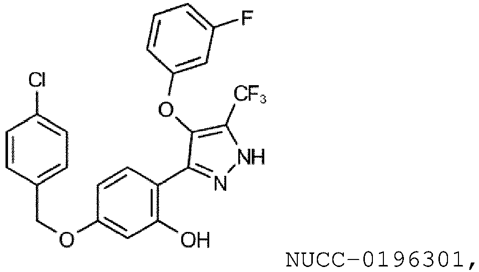 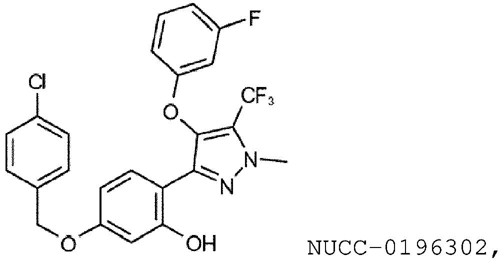 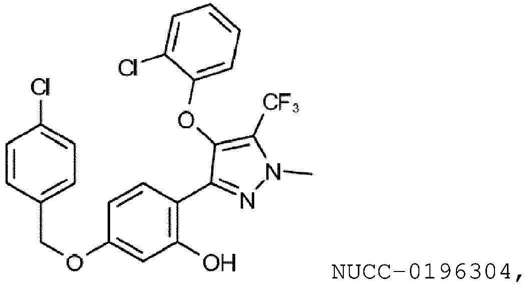 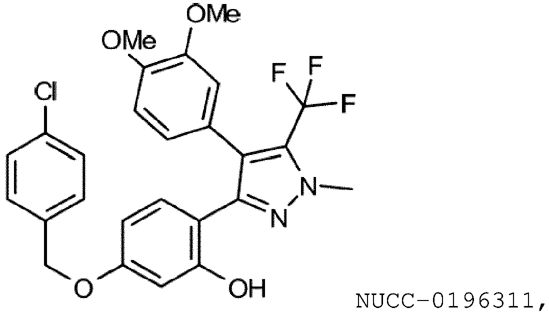 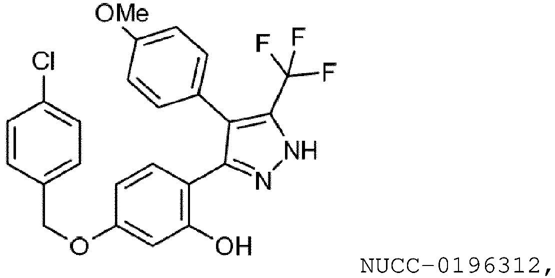 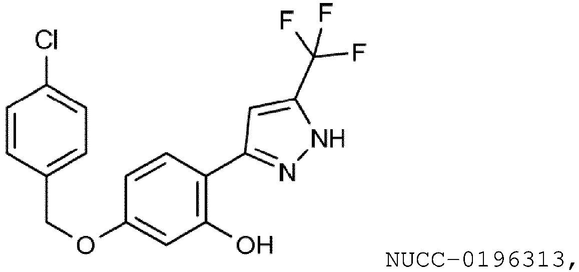 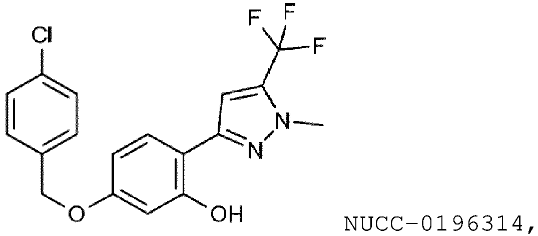 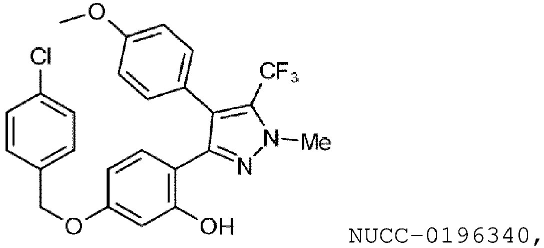 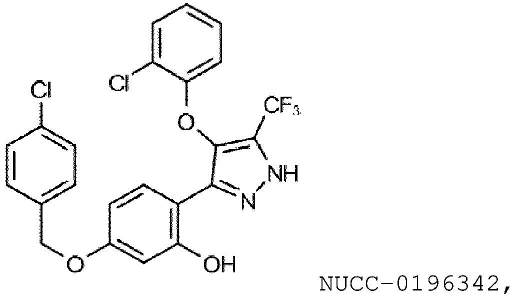 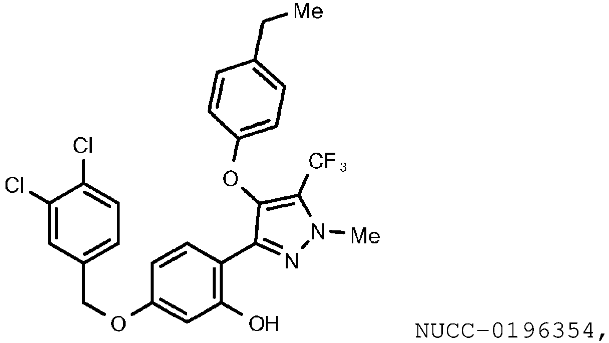 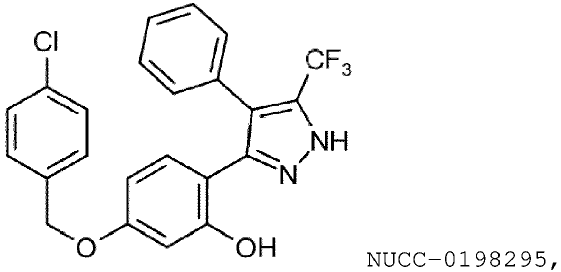 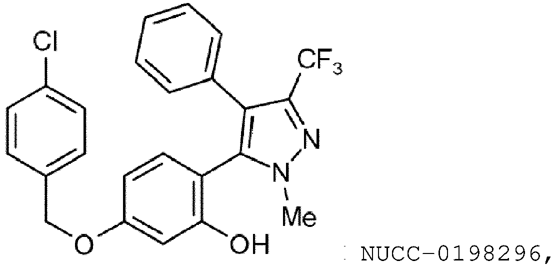 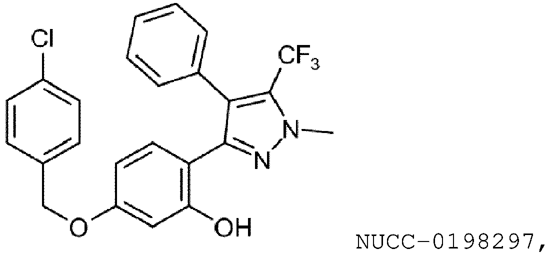 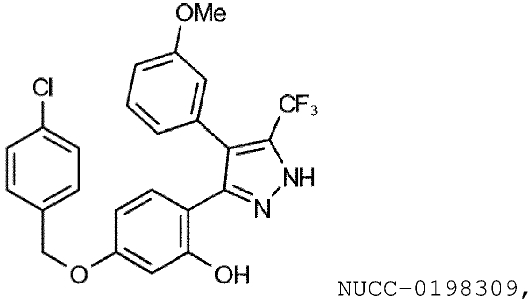 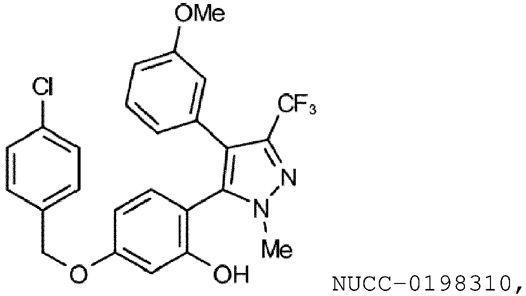 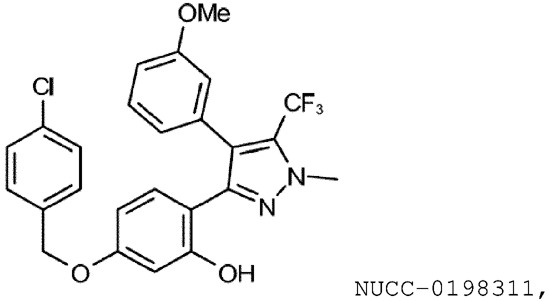 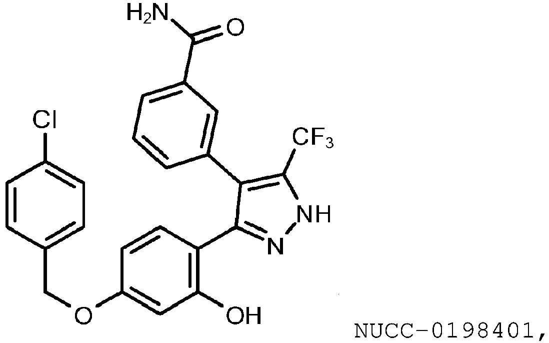 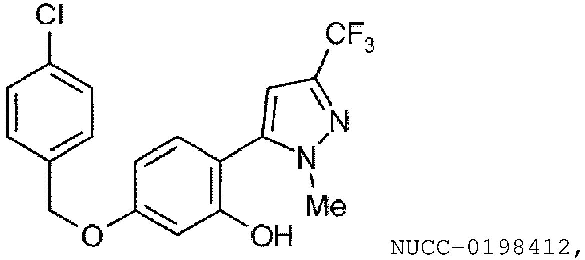 et

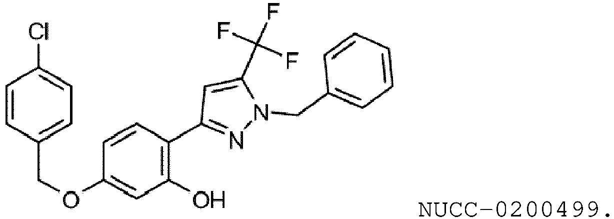

2. Composition pharmaceutique comprenant un composé selon la revendication 1 et un support, excipient ou diluant pharmaceutique approprié.

3. Composition pharmaceutique selon la revendication 2 pour une utilisation dans un procédé de traitement du cancer.
